# EUROPEAN PATENT APPLICATION

(11) **EP 4 190 810 A1**
(43) Date of publication of application: **07.06.2023**
(21) Application number: 21211717.0
(22) Date of filing: 01.12.2021
(51) Int. Cl.: C07K 16/10, A61K 39/395, C12N 15/13, A61P 31/14

(54) **NEUTRALIZING ANTIBODIES AGAINST SARS-RELATED CORONAVIRUS**

(71) Applicant: Universität zu Köln, 50923 Köln (DE)
(72) Inventor: Klein, Florian, 50937 Köln (DE); Vanshylla, Kanika, 50938 Köln (DE); Grüll, Henning, 50672 Köln (DE)
(74) Representative: Klöckner, Christoph

(57) **Abstract**

The present invention relates to antibodies or antigen-binding fragments thereof against SARS-related coronavirus, pharmaceutical compositions comprising such antibodies or antigen-binding fragments thereof, a kit comprising such antibodies or antigen-binding fragments thereof, and the antibodies or antigen-binding fragments thereof and the pharmaceutical composition and the kit for use as a medicament, and in the treatment or prevention of a disease caused by SARS-related coronavirus.

## Description

### Technical field

The present invention relates to antibodies or antigen-binding fragments thereof against SARS-related coronavirus, pharmaceutical compositions comprising such antibodies or antigen-binding fragments thereof, a kit comprising such antibodies or antigen-binding fragments thereof, and the antibodies or antigen-binding fragments thereof and the pharmaceutical compositions and the kit for use as a medicament, and in the treatment or prevention of a disease caused by SARS-related coronavirus. The present invention further relates to methods of treating, preventing or reducing the severity of an infection with a SARS-related coronavirus, and to nucleic acids encoding such antibodies or antigen-binding fragments thereof, expression vectors comprising such nucleic acids, host cells comprising such nucleic acids or expression vectors, and methods for the production of such antibodies or antigen-binding fragments thereof.

### Background of the Invention

The coronavirus disease-19 (COVID-19) pandemic has led to the loss of over 4 million lives worldwide in the last 1.5 years. Although a majority of severe acute respiratory syndrome coronavirus 2 (SARS-CoV-2) infections are mild, hospitalizations and death can occur in all age groups and older individuals with co-morbidities are at particular risk. The rapid and successful development of effective SARS-CoV-2 vaccines has been a critical breakthrough. Vaccines can protect from severe disease and death as well as mitigate the spread of infection.

However, unvaccinated individuals and vulnerable groups such as immune-deficient patients who cannot mount adequate immune responses still remain susceptible to infection and severe complications. Moreover, the current rise in SARS-CoV-2 variants with antigenic escape mutations can reduce the efficacy of approved therapeutics as well as vaccines and result in increased incidence of breakthrough infections. This warrants the need for interventions that can effectively treat SARS-CoV-2 infection by preventing severe disease and reducing morbidity.

Neutralizing antibodies (NAbs) are an important part of the humoral immune system for preventing and cleaning viral infections. They can block viral entry into cells and mediate clearance of viral particles through Fc-mediated effector functions. Advanced single B-cell cloning strategies have helped decipher the B-cell response to SARS-CoV-2 and resulted in the isolation of several potent monoclonal antibodies (mAbs) (Andreano, E. et al. (2021). Cell 184, 1821-1835 e1816; Ju, B. et al. (2020). Nature 584, 115-119; Kreer, C. et al. (2020). Cell 182, 843-854 e812; Liu, L. et al. (2020). Nature 584, 450-456; Robbiani, D.F. et al. (2020). Nature 584, 437-442; Rogers, T.F. et al. (2020). Science 369, 956-963; Zost, S.J. et al. (2020). Nat Med 26, 1422-1427.).

These SARS-CoV-2 antibodies are directed against the spike (S) protein expressed on the virus surface, which facilitates viral entry into human cells by binding to the human ACE-2 receptor. On the spike protein, a large fraction of the NAb response is directed at the receptor binding domain (RBD) or the N-terminal domain (NTD) of the SARS-CoV-2 spike S1 domain. The S2 domain of the spike protein is more conserved than the S1 amongst β-coronaviruses (β-CoVs), however, mAbs targeting the S2 domain are rare and less potent (Pinto, D. et al. (2021). Science 373, 1109-1116; Sauer, M.M. et al. (2021). Nat Struct Mol Biol 28, 478-486.).

The highly potent neutralizing capacity of RBD-directed antibodies has led to the clinical development of mAbs for treating and preventing COVID-19. Passive immunization with mAbs can prevent infection in exposed individuals as well as treat COVID-19 and prevent progression to severe disease. Several of these mAbs have been authorized for use by FDA and EMA approval for treatment of COVID-19 or are currently being investigated in phase-III clinical trials.

Despite the success in antibody-mediated treatment of SARS-CoV-2 infection, the recent emergence of SARS-CoV-2 variants with antigenic escape mutations in the spike protein has led to reduced effectiveness or rendered authorized antibodies ineffective due to loss of neutralizing activity.

Therefore, it is essential to develop next-generation mAbs that retain potency and effectiveness against circulating or emerging SARS-CoV-2 variants. There remains a demand for human antibodies directed against SARS-related coronavirus which do not show autoreactivity and have superior neutralization potency against circulating SARS-CoV-2 variants of concern as well as emerging escape variants in comparison to publicly available antibodies published in sufficient detail.

Thus, it is an object of the present invention to provide novel monoclonal antibodies against SARS-related coronavirus which do not demonstrate autoreactivity and have excellent neutralization potency against circulating SARS-CoV-2 variants of concern as well as emerging escape variants. It is a further object of the present invention to provide novel monoclonal antibodies against SARS-related coronavirus which can be used in treatment or prevention of a disease caused by SARS-related coronavirus in human or animal subjects as well as in prevention of infection of a human or animal subject with SARS-related coronavirus.

### Summary of the Invention

These objects have been solved by the aspects of the present invention as specified hereinafter.

According to the first aspect of the present invention, an antibody or antigen-binding fragment thereof directed against SARS-related coronavirus is provided, wherein the antibody or antigen-binding fragment thereof comprises the combination of the heavy chain CDR1 to CDR3 and the light chain CDR1 to CDR3 amino acid sequence of one antibody selected from the group comprising R200-1B9 (having a CDR-H1 amino acid sequence of SEQ ID No. 27, a CDR-H2 amino acid sequence of SEQ ID No. 28, a CDR-H3 amino acid sequence of SEQ ID No. 29, a CDR-L1 amino acid sequence of SEQ ID No. 30, a CDR-L2 amino acid sequence of SEQ ID No. 31, a CDR-L3 amino acid sequence of SEQ ID No. 32), R207-2F11 (having a CDR-H1 amino acid sequence of SEQ ID No. 33, a CDR-H2 amino acid sequence of SEQ ID No. 34, a CDR-H3 amino acid sequence of SEQ ID No. 35, a CDR-L1 amino acid sequence of SEQ ID No. 36, a CDR-L2 amino acid sequence of SEQ ID No. 37, a CDR-L3 amino acid sequence of SEQ ID No. 38), R40-1G8 (having a CDR-H1 amino acid sequence of SEQ ID No. 39, a CDR-H2 amino acid sequence of SEQ ID No. 40, a CDR-H3 amino acid sequence of SEQ ID No. 41, a CDR-L1 amino acid sequence of SEQ ID No. 42, a CDR-L2 amino acid sequence of SEQ ID No. 43, a CDR-L3 amino acid sequence of SEQ ID No. 44), R568-2G5 (having a CDR-H1 amino acid sequence of SEQ ID No. 45, a CDR-H2 amino acid sequence of SEQ ID No. 46, a CDR-H3 amino acid sequence of SEQ ID No. 47, a CDR-L1 amino acid sequence of SEQ ID No. 48, a CDR-L2 amino acid sequence of SEQ ID No. 49, a CDR-L3 amino acid sequence of SEQ ID No. 50), R568-2B11 (having a CDR-H1 amino acid sequence of SEQ ID No. 51, a CDR-H2 amino acid sequence of SEQ ID No. 52, a CDR-H3 amino acid sequence of SEQ ID No. 53, a CDR-L1 amino acid sequence of SEQ ID No. 54, a CDR-L2 amino acid sequence of SEQ ID No. 55, a CDR-L3 amino acid sequence of SEQ ID No. 56), R207-2G4 (having a CDR-H1 amino acid sequence of SEQ ID No. 57, a CDR-H2 amino acid sequence of SEQ ID No. 58, a CDR-H3 amino acid sequence of SEQ ID No. 59, a CDR-L1 amino acid sequence of SEQ ID No. 60, a CDR-L2 amino acid sequence of SEQ ID No. 61, a CDR-L3 amino acid sequence of SEQ ID No. 62), R40-1C8 (having a CDR-H1 amino acid sequence of SEQ ID No. 63, a CDR-H2 amino acid sequence of SEQ ID No. 64, a CDR-H3 amino acid sequence of SEQ ID No. 65, a CDR-L1 amino acid sequence of SEQ ID No. 66, a CDR-L2 amino acid sequence of SEQ ID No. 67, a CDR-L3 amino acid sequence of SEQ ID No. 68), R568-2B9 (having a CDR-H1 amino acid sequence of SEQ ID No. 69, a CDR-H2 amino acid sequence of SEQ ID No. 70, a CDR-H3 amino acid sequence of SEQ ID No. 71, a CDR-L1 amino acid sequence of SEQ ID No. 72, a CDR-L2 amino acid sequence of SEQ ID No. 73, a CDR-L3 amino acid sequence of SEQ ID No. 74), R568-1B3 (having a CDR-H1 amino acid sequence of SEQ ID No. 75, a CDR-H2 amino acid sequence of SEQ ID No. 76, a CDR-H3 amino acid sequence of SEQ ID No. 77, a CDR-L1 amino acid sequence of SEQ ID No. 78, a CDR-L2 amino acid sequence of SEQ ID No. 79, a CDR-L3 amino acid sequence of SEQ ID No. 80), R568-2E1 (having a CDR-H1 amino acid sequence of SEQ ID No. 81, a CDR-H2 amino acid sequence of SEQ ID No. 82, a CDR-H3 amino acid sequence of SEQ ID No. 83, a CDR-L1 amino acid sequence of SEQ ID No. 84, a CDR-L2 amino acid sequence of SEQ ID No. 85, a CDR-L3 amino acid sequence of SEQ ID No. 86), R568-1G9 (having a CDR-H1 amino acid sequence of SEQ ID No. 87, a CDR-H2 amino acid sequence of SEQ ID No. 88, a CDR-H3 amino acid sequence of SEQ ID No. 89, a CDR-L1 amino acid sequence of SEQ ID No. 90, a CDR-L2 amino acid sequence of SEQ ID No. 91, a CDR-L3 amino acid sequence of SEQ ID No. 92), R121-1F1 (having a CDR-H1 amino acid sequence of SEQ ID No. 93, a CDR-H2 amino acid sequence of SEQ ID No. 94, a CDR-H3 amino acid sequence of SEQ ID No. 95, a CDR-L1 amino acid sequence of SEQ ID No. 96, a CDR-L2 amino acid sequence of SEQ ID No. 97, a CDR-L3 amino acid sequence of SEQ ID No. 98), R259-1B9 (having a CDR-H1 amino acid sequence of SEQ ID No. 99, a CDR-H2 amino acid sequence of SEQ ID No. 100, a CDR-H3 amino acid sequence of SEQ ID No. 101, a CDR-L1 amino acid sequence of SEQ ID No. 102, a CDR-L2 amino acid sequence of SEQ ID No. 103, a CDR-L3 amino acid sequence of SEQ ID No. 104).

In one embodiment of the first aspect of the present invention, the antibody or antigen-binding fragment thereof comprises the combination of the variable region heavy chain amino acid sequence and of the variable region light chain amino acid sequence of one antibody selected from the group comprising R200-1B9 (having the variable region heavy chain amino acid sequence of SEQ ID No. 1 and the variable region light chain amino acid sequence of SEQ ID No. 2), R207-2F11 (having the variable region heavy chain amino acid sequence of SEQ ID No. 3 and the variable region light chain amino acid sequence of SEQ ID No. 4), R40-1G8 (having the variable region heavy chain amino acid sequence of SEQ ID No. 5 and the variable region light chain amino acid sequence of SEQ ID No. 6), R568-2G5 (having the variable region heavy chain amino acid sequence of SEQ ID No. 7 and the variable region light chain amino acid sequence of SEQ ID No. 8), R568-2B11 (having the variable region heavy chain amino acid sequence of SEQ ID No. 9 and the variable region light chain amino acid sequence of SEQ ID No. 10), R207-2G4 (having the variable region heavy chain amino acid sequence of SEQ ID No. 11 and the variable region light chain amino acid sequence of SEQ ID No. 12), R40-1C8 (having the variable region heavy chain amino acid sequence of SEQ ID No. 13 and the variable region light chain amino acid sequence of SEQ ID No. 14), R568-2B9 (having the variable region heavy chain amino acid sequence of SEQ ID No. 15 and the variable region light chain amino acid sequence of SEQ ID No. 16), R568-1B3 (having the variable region heavy chain amino acid sequence of SEQ ID No. 17 and the variable region light chain amino acid sequence of SEQ ID No. 18), R568-2E1 (having the variable region heavy chain amino acid sequence of SEQ ID No. 19 and the variable region light chain amino acid sequence of SEQ ID No. 20), R568-1G9 (having the variable region heavy chain amino acid sequence of SEQ ID No. 21 and the variable region light chain amino acid sequence of SEQ ID No. 22), R121-1F1 (having the variable region heavy chain amino acid sequence of SEQ ID No. 23 and the variable region light chain amino acid sequence of SEQ ID No. 24), R259-1B9 (having the variable region heavy chain amino acid sequence of SEQ ID No. 25 and the variable region light chain amino acid sequence of SEQ ID No. 26).

According to one embodiment of the first aspect of the present invention, the amino acid sequences comprised are of one antibody selected from the group comprising R200-1B9, R207-2F11, R40-1G8, R568-2G5, R568-2B11, R207-2G4, R40-1C8, R568-2B9, and R568-1B3, preferably of one antibody from the group comprising R200-1B9, R207-2F11, R40-1G8, and R568-2G5.

According to another embodiment of the first aspect of the present invention, the SARS-related coronavirus strain is severe acute respiratory syndrome coronavirus 2 (SARS-CoV-2).

According to yet another embodiment of the first aspect of the present invention, the amino acid sequences of the CDRs or of the variable regions comprised in the antibody or antigen-binding fragment thereof are taken from an antibody which is able to neutralize each of the SARS-CoV-2 lineages B.1, B.1.1.7, B.1.351, B.1.429, B.1.617 and B.1.617.2 in a pseudovirus neutralization assay as described in the description with an IC50 of at most 0.4 µg/ml, preferably at most 0.1 µg/ml, more preferably at most 0.05 µg/ml, even more preferably at most 0.025 µg/ml, even more preferably at most 0.01 µg/ml, even more preferably at most 0.0025 µg/ml, particularly preferably at most 0.0015 µg/ml.

According to yet another embodiment of the first aspect of the present invention, the amino acid sequences of the CDRs or of the variable regions comprised in the antibody or antigen-binding fragment thereof are taken from an antibody which is able to neutralize each of the SARS-CoV-2 spike escape mutants 69-70del, K417E, N439K, K444Q, V445A, G446V, L452R, Y453F, L455F, G476S, S477N, E484K, F486V, F490S, Q493R, Q493K, S494P, N501Y in a pseudovirus neutralization assay as described in the description with an IC50 of at most 1.3 µg/ml, preferably at most 0.5 µg/ml, more preferably at most 0.05 µg/ml, even more preferably at most 0.02 µg/ml, particularly preferably at most 0.01 µg/ml.

According to one embodiment of the first aspect of the present invention, the amino acid sequences of the CDRs or of the variable regions comprised in the antibody or antigen-binding fragment thereof are taken from an antibody which is able to neutralize each of the emerging SARS-CoV-2 escape variants R346S, Q414H, N440K, and T478K in a pseudovirus neutralization assay as described in the description with an IC50 of at most 0.0015 µg/ml.

According to yet another embodiment of the first aspect of the present invention, the amino acid sequences of the CDRs or of the variable regions comprised in the antibody or antigen-binding fragment thereof are taken from an antibody which is able to neutralize the SARS-CoV-2 variants Wu-01, B.1, B.1.1.7, B.1.351, B.1.429, B.1.617 and B.1.617.2 and the SARS-CoV-2 escape mutants 69-70del, K417E, N439K, K444Q, V445A, G446V, L452R, Y453F, L455F, G476S, S477N, E484K, F486V, F490S, Q493R, Q493K, S494P, N501Y, R346S, Q414H, N440K, and T478K in a pseudovirus neutralization assay as described in the description across all variants and mutants with an average IC50 of at most 0.08 µg/ml, preferably of at most 0.025 µg/ml, more preferably of at most 0.01 µg/ml, even more preferably of at most 0.006 µg/ml, particularly preferably of at most 0.002 µg/ml.

According to another embodiment of the present invention, the antibody or antigen-binding fragment thereof does not display autoreactivity defined as a detectable binding pattern when tested against permeabilized HEp-2 cells using an antinuclear antibody (ANA) testing kit (NOVA-Lite HEp-2 ANA kit; Inova Diagnostics) at concentrations of 100 µg/ml of the antibody or antigen-binding fragment thereof.

According to the second aspect of the present invention, a pharmaceutical composition is provided comprising an antibody or antigen-binding fragment thereof according to the first aspect of the present invention, and at least one pharmaceutically acceptable excipient.

According to an embodiment of the second aspect of the present invention, the pharmaceutical composition is a vaccination composition for a human or animal subject.

According to the third aspect of the present invention, a kit is provided comprising an antibody or antigen-binding fragment thereof according to the first aspect of the present invention, and a container.

According to the fourth aspect of the present invention, an antibody or antigen-binding fragment thereof according to the first aspect of the invention, a pharmaceutical composition according to the second aspect of the invention, or a kit according to the third aspect of the invention are provided for use as a medicament, preferably for use as a vaccine.

According to the fifth aspect of the present invention, an antibody or antigen-binding fragment thereof according to the first aspect of the invention, a pharmaceutical composition according to the second aspect of the invention, or a kit according to the third aspect of the invention are provided for use in the treatment or prevention of a disease caused by SARS-related coronavirus in human or animal subjects, preferably for use in the treatment or prevention of a disease caused by severe acute respiratory syndrome coronavirus 2 (SARS-CoV-2) in human or animal subjects.

According to the sixth aspect of the present invention, an antibody or antigen-binding fragment thereof according to the first aspect of the invention, a pharmaceutical composition according to the second aspect of the invention, or a kit according to the third aspect of the invention are provided for use in prevention of infection of a human or animal subject with SARS-related coronavirus, preferably of infection of a human or animal subject with severe acute respiratory syndrome coronavirus 2 (SARS-CoV-2).

According to the seventh aspect of the present invention, a method of treating or preventing a SARS-related coronavirus infection or reducing the severity of disease in a human and/or animal subject is provided comprising administering a therapeutically effective amount of at least one antibody and/or antigen-binding fragment thereof according to the first aspect of the invention to said subject.

According to an embodiment of the fourth aspect or of the fifth aspect or of the sixth aspect or of the seventh aspect of the present invention, the antibody is provided in combination with at least one further antibody directed against SARS-related coronavirus 2 (SARS-CoV-2), wherein said further antibody has a different binding specificity.

According to an embodiment of the fourth aspect or of the fifth aspect or of the sixth aspect or of the seventh aspect of the present invention, the antibody is administered by intravenous infusion, by subcutaneous injection, by intramuscular injection or by inhalative application.

In one embodiment of the fourth aspect or of the fifth aspect or of the sixth aspect or of the seventh aspect of the present invention, the antibody or antigen-binding fragment of the present invention is administered at an absolute dose of up to 4000 mg, preferably up to 2400 mg, more preferably up to 1200 mg, even more preferably up to 600 mg, even more preferably up to 300 mg, particularly preferably up to 150 mg.

According to the eighth aspect of the present invention, a nucleic acid is provided encoding an antibody or antigen-binding fragment thereof according to the first aspect of the present invention.

According to the ninth aspect of the present invention, an expression vector is provided comprising the nucleic acid according to the eighth aspect of the present invention in functional association with an expression control sequence.

According to the tenth aspect of the present invention, a host cell is provided comprising a nucleic acid according to the eighth aspect of the present invention or the expression vector according to the ninth aspect of the present invention.

According to the eleventh aspect of the present invention, a method of production of an antibody or antigen-binding fragment thereof according to the first aspect of the present invention is provided, comprising cultivating the host cell according to the tenth aspect of the present invention under conditions allowing expression of the antibody or antigen-binding fragment thereof, and recovering the antibody or antigen-binding fragment thereof.

### Description of Figures

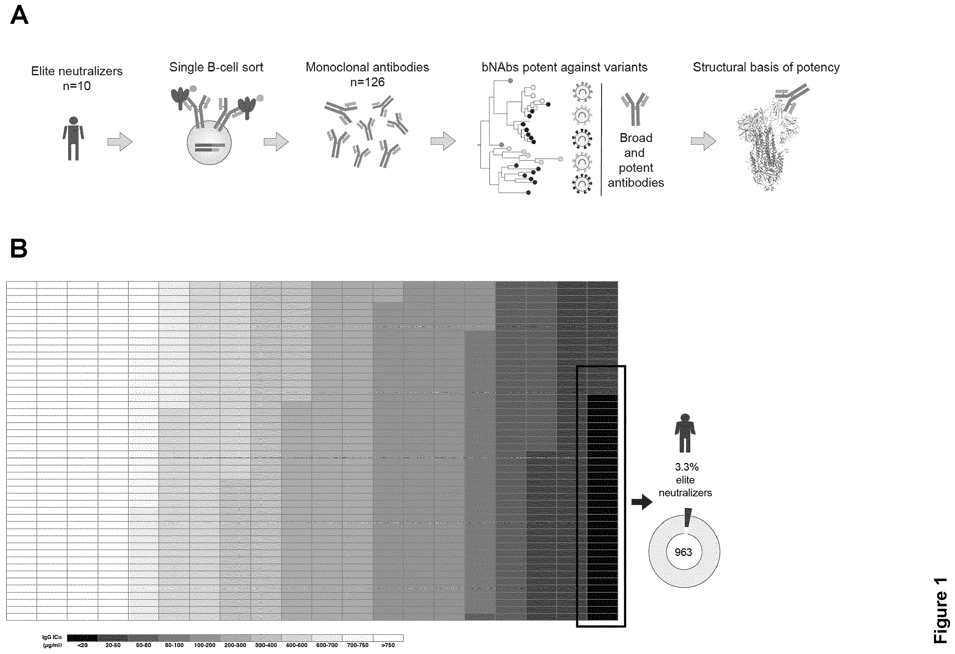

Figure 1 shows (A) a schematic of study design used to identify and isolate monoclonal antibodies from SARS-CoV-2 elite neutralizers; (B) a heat map depicting the serum or plasma IgG neutralization IC₅₀ values against the SARS-CoV-2 Wu01 pseudovirus in the COVID-19 convalescent cohort studied (Vanshylla, K. et al. (2021). Cell Host Microbe 29, 917-929); the pie chart shows the fraction (3.3%) of elite neutralizers in the cohort.

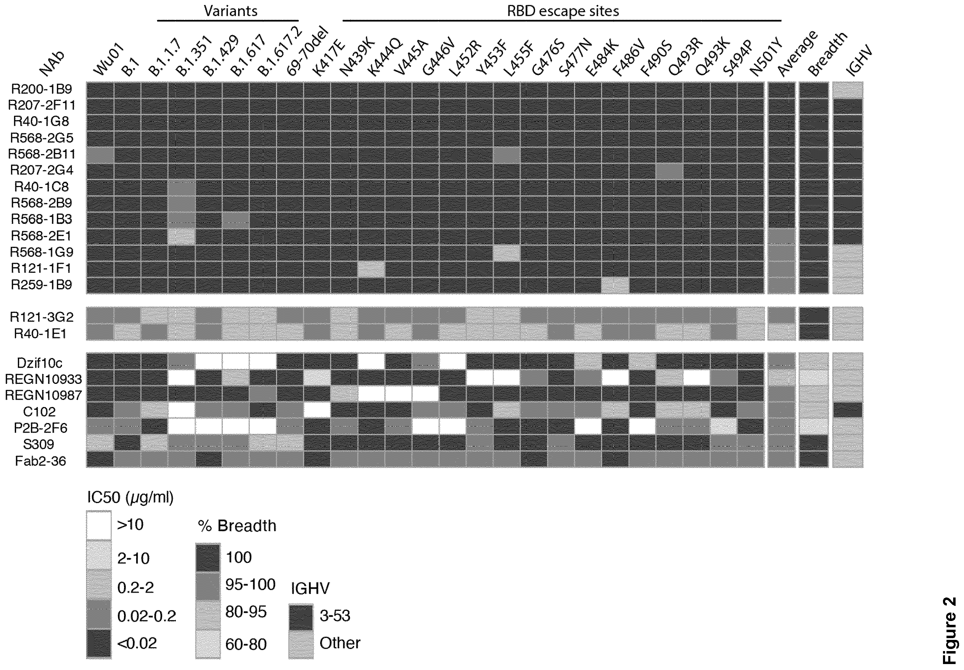

Figure 2 shows the neutralization profile of the elite neutralizer NAbs of the invention (top panel) and reference antibodies (bottom panel) against 25 SARS-CoV-2 spike pseudovirus variants; average IC₅₀ values and relative neutralization breadth across the variants, as well as IGHV3-53 usage are depicted in columns to the right; cross-neutralizing antibodies R121-3G2 and R40-1E1 with neutralizing activity against a SARS-CoV-1 and the Bat-CoV WIV-1 pseudovirus are shown in the center panel.

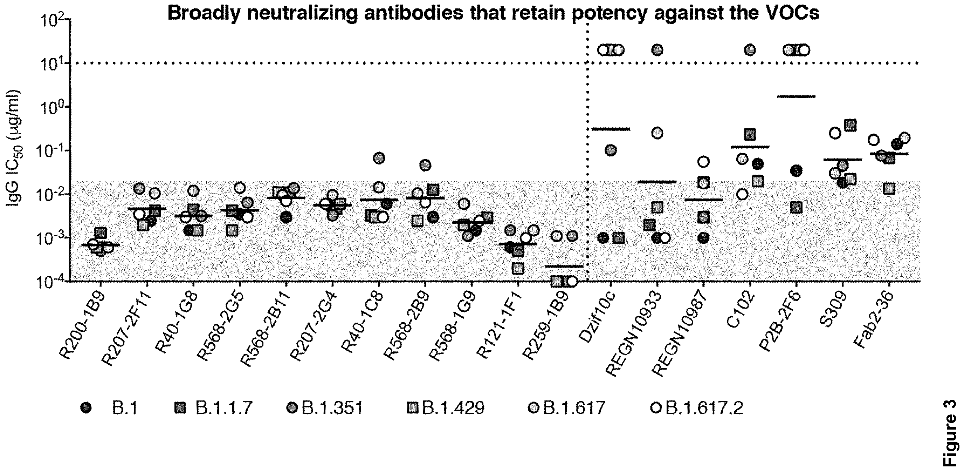

Figure 3 shows a plot indicating the IC₅₀ values of the broadest (100%) and most potent elite Nabs of the present invention against SARS-CoV-2 pseudovirus variants with spike sequence of the circulating variants B.1, B.1.1.7, B.1.351, B.1.429, B.1.617 and B.1.617.2 compared to published monoclonal reference antibodies including antibodies authorized for clinical use; grey area highlights values below 0.02 µg/ml, and lines indicate geometric mean IC₅₀ values across all variants.

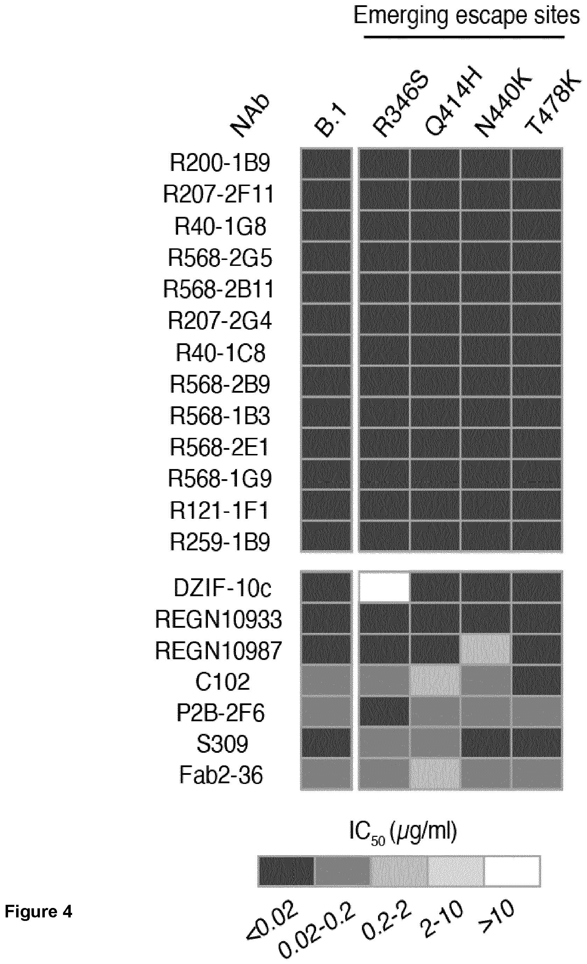

Figure 4 shows a neutralization analysis of the 13 most potent and broad bNAbs of the present invention along with published antibodies tested against SARS-CoV-2 pseudoviruses carrying one of the four emerging spike mutations, R346S, Q414H, N440K and T478K.

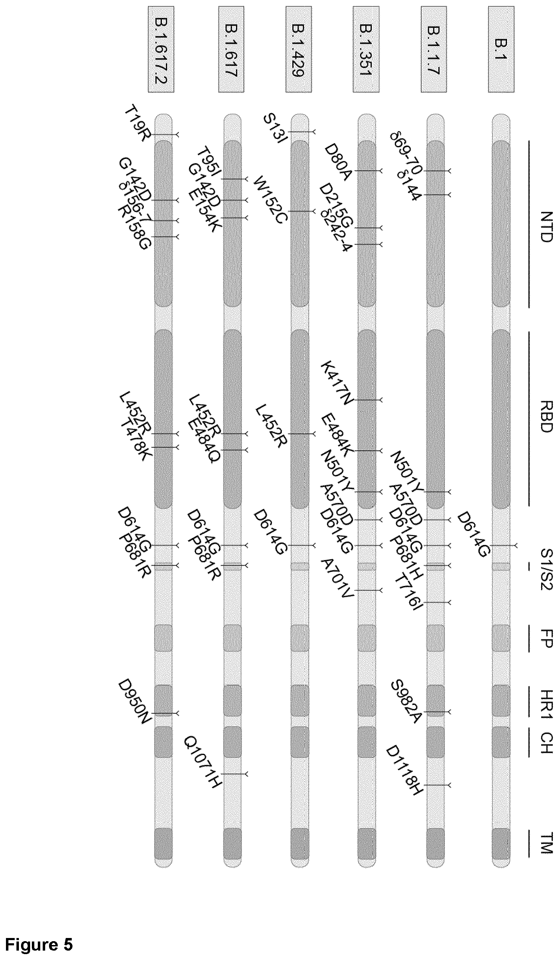

Figure 5 shows a schematic of the SARS-COV-2 spike domains highlighting the residues mutated in the VOCs or VOIs used in the study.

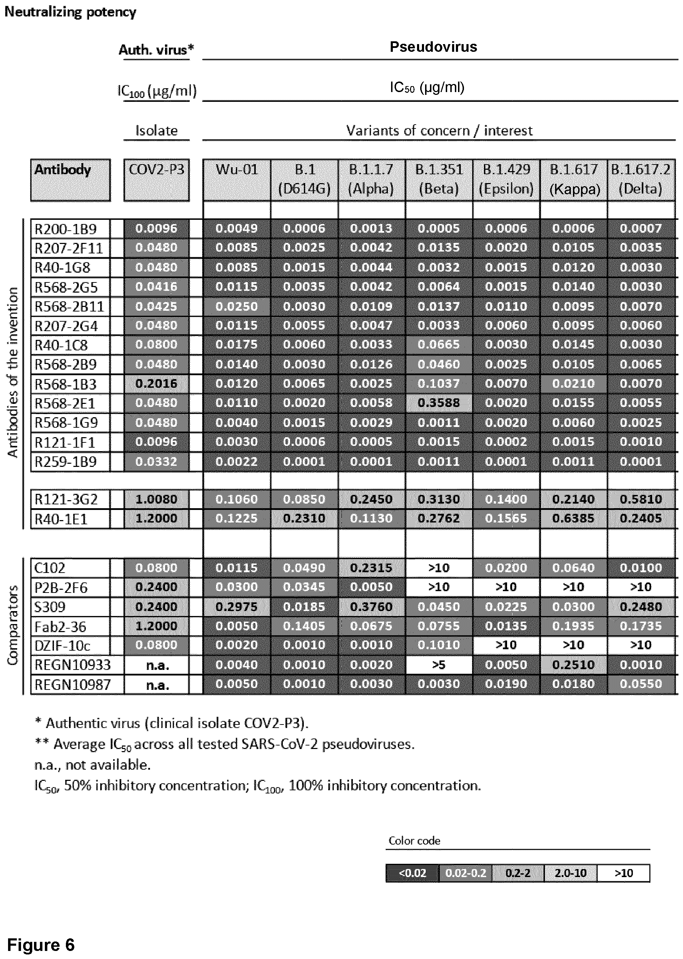

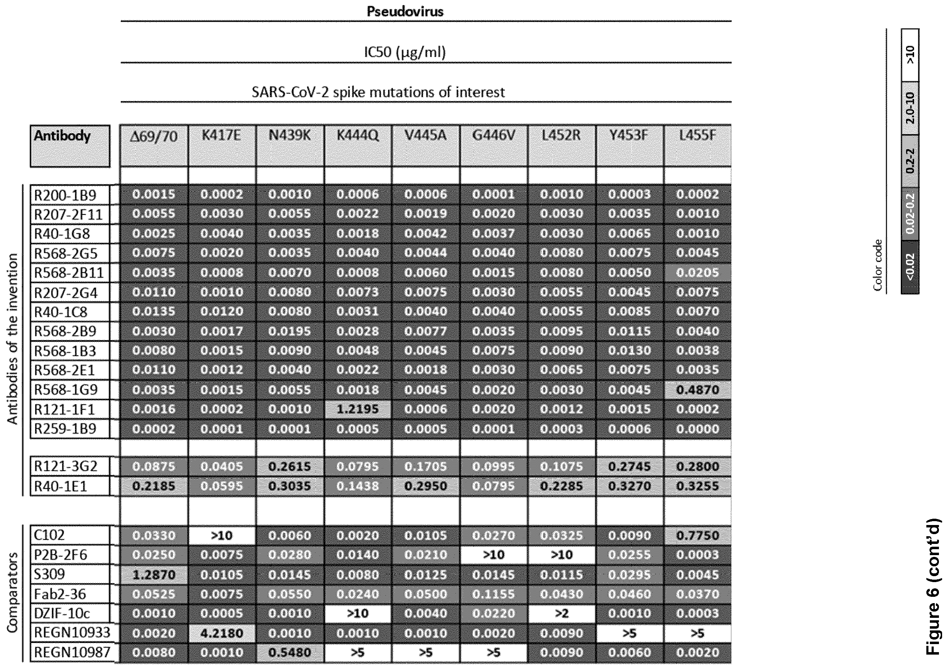

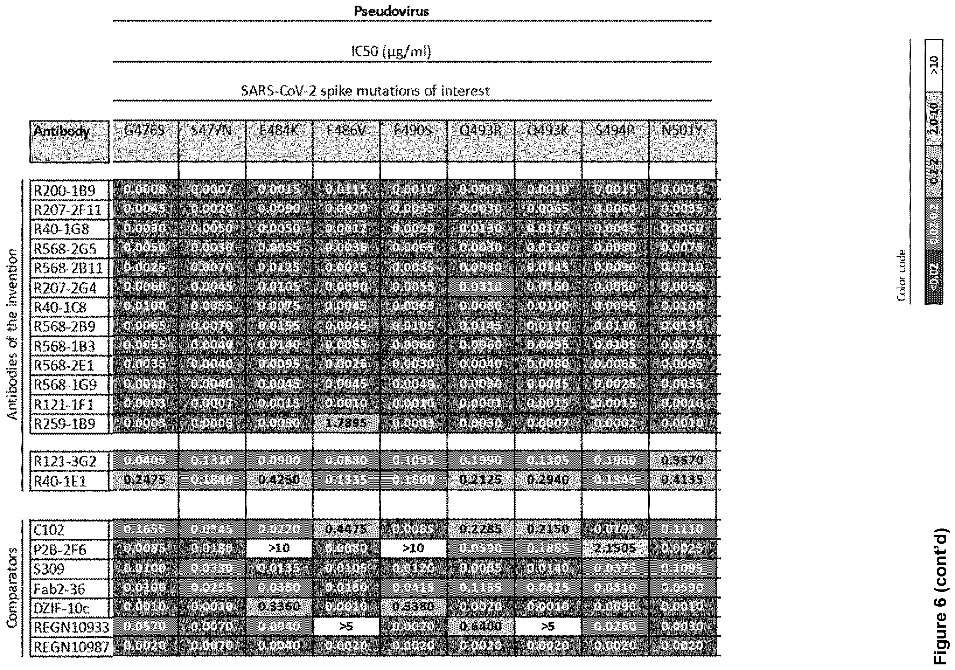

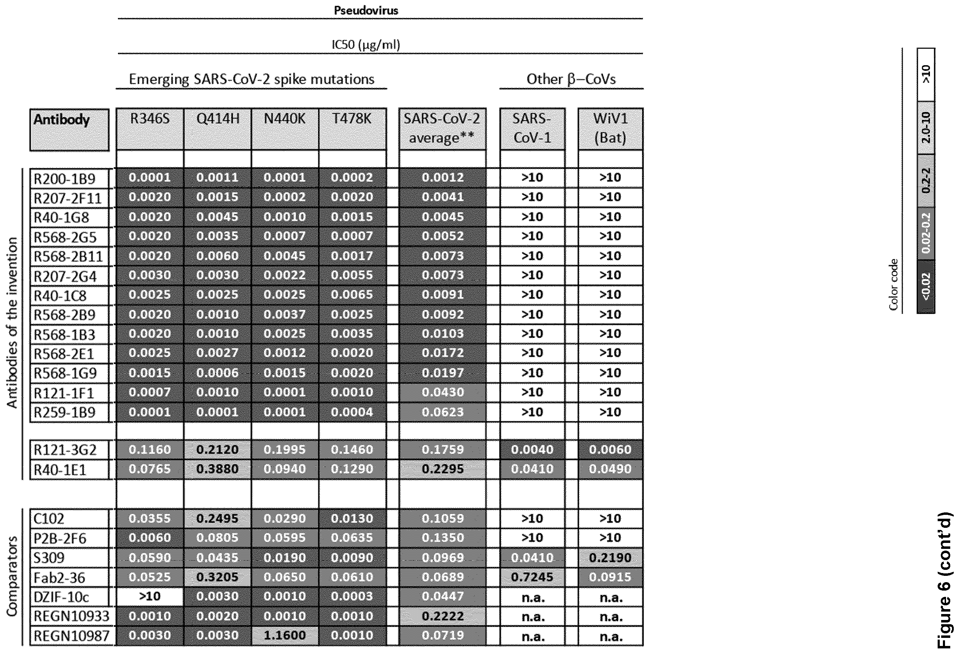

Figure 6 shows individual values for the neutralizing potency of the 13 most potent and broad bNAbs of the present invention as well as the cross-neutralizing antibodies R121-3G2 and R40-1E1 of the present invention along with published antibodies tested against an authentic virus isolate (indicated as IC₁₀₀ in µg/ml), and against 25 SARS-CoV-2 spike pseudovirus variants as illustrated in Figure 2 as well as against SARS-CoV-2 pseudovirus variants carrying one of the four emerging spike mutations, R346S, Q414H, N440K and T478K as illustrated in Figure 4 (indicated as IC₅₀ in µg/ml); the average neutralization potency across all 25 tested SARS-CoV-2 pseudovirus variants, as well as neutralizing potency against SARS-CoV-1 pseudovirus and against a WIV-1 (bat coronavirus) pseudovirus are additionally displayed.

### Detailed Description of the Invention

In order that the present description can be more readily understood, certain terms are first defined. Additional definitions are set forth throughout the detailed description.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International d'Unités (SI) accepted form. Numeric ranges are inclusive of the numbers defining the range. Unless otherwise indicated, nucleotide sequences are written left to right in 5' to 3' orientation. Amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower).

The term "antibody" is used herein in the broadest sense to refer to molecules with an immunoglobulin-like domain (for example IgG, IgM, IgA, IgD or IgE) and includes monoclonal, recombinant, polyclonal, chimeric, human, humanized, multispecific antibodies, including bispecific antibodies, and heteroconjugate antibodies; a single variable domain (e.g., V_{H}, V_{HH}, V_{L}, domain antibody), antigen binding antibody fragments, Fab, F(ab')₂, Fv, disulphide linked Fv, single chain Fv, disulphide-linked scFv, diabodies, etc. and modified versions of any of the foregoing.

The term "antibody" as used herein refers to a protein, derived from a germline immunoglobulin sequence, which is capable of specifically binding to an antigen or an antigen-binding portion thereof. The term includes full length antibodies of any class or isotype (that is, IgA, IgD, IgE, IgG, IgM and/or IgY) and any single chain or fragment thereof. An antibody that specifically binds to an antigen, or antigen-binding portion thereof, may bind exclusively to that antigen, or portion thereof, or it may bind to a limited number of homologous antigens, or portions thereof. Full-length antibodies usually comprise at least four polypeptide chains: two heavy (H) chains and two light (L) chains that are interconnected by disulfide bonds.

One immunoglobulin sub-class of particular pharmaceutical interest is the IgG family. In humans, the IgG class may be sub-divided into 4 sub-classes: IgG₁, IgG₂, IgG₃ and IgG₄, based on the sequence of their heavy chain constant regions. The light chains can be divided into two types, kappa and lambda, based on differences in their sequence composition. IgG molecules are composed of two heavy chains, interlinked by two or more disulfide bonds, and two light chains, each attached to a heavy chain by a disulfide bond. A heavy chain may comprise a heavy chain variable region (VH) and up to three heavy chain constant (CH) regions: CH1, CH2 and CH3. A light chain may comprise a light chain variable region (VL) and a light chain constant region (CL).

VH and VL regions can be further subdivided into regions of hypervariability, termed complementarity determining regions (CDRs), interspersed with regions that are more conserved, termed framework regions (FR). VH and VL regions are typically composed of three CDRs and four FRs, arranged from amino-terminus to carboxy-terminus in the following order: FR1, CDR1, FR2, CDR2, FR3, CDR3, FR4. The hypervariable regions of the heavy and light chains form a binding domain that is capable of interacting with an antigen, while the constant region of an antibody may mediate binding of the immunoglobulin to host tissues or factors, including but not limited to various cells of the immune system (effector cells), Fc receptors and the first component (C1q) of the classical complement system. Antibodies of the current invention may be isolated.

The term "isolated antibody" refers to an antibody that has been separated and/or recovered from (an)other component(s) in the environment in which it was produced and/or that has been purified from a mixture of components present in the environment in which it was produced. Certain antigen-binding fragments of antibodies may be suitable in the context of the current invention, as it has been shown that the antigen-binding function of an antibody can be performed by fragments of a full-length antibody.

The term "antigen-binding portion", "binding fragment" or "antigen-binding fragment" of an antibody refers to one or more fragment(s) of an antibody that retain the ability to specifically bind to an antigen, such as the spike (S) protein of SARS-CoV-2, as described herein.

Examples of antigen-binding fragments include Fab, Fab', F(ab)2, F(ab')2, F(ab)S, Fv (typically the VL and VH domains of a single arm of an antibody), single-chain Fv (scFv; see, e.g., Bird et al., Science 242:42S-426 (1988); Huston et al., PNAS 85: 5879-5883 (1988)), dsFv, Fd (typically the VH and CH1 domain), and dAb (typically a V_{H} domain) fragments; V_{H}, V_{L}, V_{HH}, and V-NAR domains; monovalent molecules comprising a single V_{H} and a single V_{L} chain; minibodies, diabodies, triabodies, tetrabodies, and kappa bodies (see, e.g., III et al., Protein Eng 10:949-57 (1997)); camel IgG; IgNAR; as well as one or more isolated CDRs or a functional paratope, where the isolated CDRs or antigen-binding residues or polypeptides can be associated or linked together so as to form a functional antibody fragment.

Various types of antibody fragments have been described or reviewed in, e.g., Holliger and Hudson, Nat Biotechnol 2S:1126-1136 (2005); International Publ. No. WO 2005/040219, and U.S. Publ. Nos. 2005/0238646 and 2002/0161201. These antibody fragments may be obtained using conventional techniques known to those of skill in the art, and the fragments may be screened for utility in the same manner as intact antibodies.

A "human" antibody (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The SARS-CoV-2 antibodies described herein can include amino acid residues not encoded by human germline immunoglobulin sequences (e.g., mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences. The terms "human" antibodies and "fully human" antibodies are used synonymously.

A "humanized" antibody refers to a human/non-human chimeric antibody that contains one or more sequences (CDR regions or parts thereof) that are derived from a non-human immunoglobulin. A humanized antibody is, thus, a human immunoglobulin (recipient antibody) in which at least residues from a hyper-variable region of the recipient are replaced by residues from a hyper-variable region of an antibody from a non-human species (donor antibody) such as from a mouse, rat, rabbit or non-human primate, which have the desired specificity, affinity, sequence composition and functionality. In some instances, FR residues of the human immunoglobulin are replaced by corresponding non-human residues. An example of such a modification is the introduction of one or more so-called back-mutations, which are typically amino acid residues derived from the donor antibody.

Humanization of an antibody may be carried out using recombinant techniques known to the person skilled in the art (see, e.g., Antibody Engineering, Methods in Molecular Biology, vol. 248, edited by Benny K. C. Lo). A suitable human recipient framework for both the light and heavy chain variable domain may be identified by, for example, sequence or structural homology. Alternatively, fixed recipient frameworks may be used, e.g., based on knowledge of structure, biophysical and biochemical properties. The recipient frameworks can be germline derived or derived from a mature antibody sequence. CDR regions from the donor antibody can be transferred by CDR grafting.

The CDR grafted humanized antibody can be further optimized for e.g. affinity, functionality and biophysical properties by identification of critical framework positions where re-introduction (backmutation) of the amino acid residue from the donor antibody has beneficial impact on the properties of the humanized antibody. In addition to donor antibody derived backmutations, the humanized antibody can be engineered by introduction of germline residues in the CDR or framework regions, elimination of immunogenic epitopes, site-directed mutagenesis, affinity maturation, etc.

Furthermore, humanized antibodies can comprise residues that are not found in the recipient antibody or in the donor antibody. These modifications are made to further refine antibody performance. In general, a humanized antibody will comprise at least one--typically twovariable domains, in which all or substantially all of the CDR regions correspond to those of a non-human immunoglobulin and in which all or substantially all of the FR residues are those of a human immunoglobulin sequence. The humanized antibody can, optionally, also comprise at least a portion of an immunoglobulin constant region (Fc), typically that of a human immunoglobulin. The term "humanized antibody derivative" refers to any modified form of the humanized antibody, such as a conjugate of the antibody and another agent or antibody.

The term "recombinant human antibody," as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences.

Such recombinant human antibodies comprise variable and constant regions that utilize particular human germline immunoglobulin sequences are encoded by the germline genes, but include subsequent rearrangements and mutations which occur, for example, during antibody maturation. As known in the art (see, e.g., Lonberg Nature Biotech. 23(9): 1117-1125 (2005)), the variable region contains the antigen binding domain, which is encoded by various genes that rearrange to form an antibody specific for a foreign antigen. In addition to rearrangement, the variable region can be further modified by multiple single amino acid changes (referred to as somatic mutation or hypermutation) to increase the affinity of the antibody to the foreign antigen. The constant region will change in further response to an antigen (i.e., isotype switch).

Therefore, the rearranged and somatically mutated nucleic acid molecules that encode the light chain and heavy chain immunoglobulin polypeptides in response to an antigen cannot have sequence identity with the original nucleic acid molecules, but instead will be substantially identical or similar (i.e., have at least 80% identity).

A "chimeric antibody" refers to an antibody in which the variable regions are derived from one species and the constant regions are derived from another species, such as an antibody in which the variable regions are derived from a mouse antibody and the constant regions are derived from a human antibody.

Alternative antibody formats include alternative scaffolds in which the one or more CDRs of the antigen-binding portion can be arranged onto a suitable non-immunoglobulin protein scaffold or skeleton, such as an affibody, a SpA scaffold, an LDL receptor class A domain, an avimer or an EGF domain.

The term "domain" refers to a folded protein structure which retains its tertiary structure independent of the rest of the protein. Generally, domains are responsible for discrete functional properties of proteins and in many cases may be added, removed or transferred to other proteins without loss of function of the remainder of the protein and/or of the domain.

The term "single variable domain" refers to a folded polypeptide domain comprising sequences characteristic of antibody variable domains. It, therefore, includes complete antibody variable domains such as V_{H}, V_{HH} and V_{L} and modified antibody variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

A single variable domain is capable of binding an antigen or epitope independently of a different variable region or domain. A "domain antibody" or "dAb^{™}" may be considered the same as a "single variable domain". A single variable domain may be a human single variable domain, but also includes single variable domains from other species such as rodent nurse shark and Camelid V_{HH} dAbs^{™}. Camelid V_{HH} are immunoglobulin single variable domain polypeptides that are derived from species including camel, llama, alpaca, dromedary, and guanaco, which produce heavy chain antibodies naturally devoid of light chains. Such V_{HH} domains may be humanized according to standard techniques available in the art, and such domains are considered to be "single variable domains". As used herein V_{H} includes camelid V_{HH} domains.

An antigen-binding fragment may be provided by means of arrangement of one or more CDRs on non-antibody protein scaffolds. "Protein Scaffold" as used herein includes but is not limited to an immunoglobulin (Ig) scaffold, for example an IgG scaffold, which may be a four chain or two chain antibody, or which may comprise only the Fc region of an antibody, or which may comprise one or more constant regions from an antibody, which constant regions may be of human or primate origin, or which may be an artificial chimera of human and primate constant regions.

As used herein, "isotype" refers to the antibody class (e.g., IgG1, IgG₂, IgG3, IgG4, IgM, IgA1, IgA2, IgD, and IgE antibody) that is encoded by the heavy chain constant region genes.

"Allotype" refers to naturally occurring variants within a specific isotype group, which variants differ in a few amino acids (see, e.g., Jefferis et al., mAbs 1:1 (2009)).

The phrases "an antibody recognizing an antigen" and "an antibody specific for an antigen" are used interchangeably herein with the term "an antibody which binds specifically to an antigen."

By the terms "treat," "treating," or "treatment of" (or grammatically equivalent terms) it is meant that the severity of the subject's condition is reduced or at least partially improved or ameliorated and/or that some alleviation, mitigation or decrease in at least one clinical symptom is achieved and/or there is a delay in the progression of the condition.

As used herein, the terms "prevent," "prevents," or "prevention" and "inhibit," "inhibits," or "inhibition" (and grammatical equivalents thereof) are not meant to imply complete abolition of disease and encompasses any type of prophylactic treatment that reduces the incidence of the condition, delays the onset of the condition, and/or reduces the symptoms associated with the condition after onset.

An "effective," "prophylactically effective," or "therapeutically effective" amount as used herein is an amount that is sufficient to provide some improvement or benefit to the subject. Alternatively stated, an "effective," "prophylactically effective," or "therapeutically effective" amount is an amount that will provide some delay, alleviation, mitigation, or decrease in at least one clinical symptom in the subject. Those skilled in the art will appreciate that the effects need not be complete or curative, as long as some benefit is provided to the subject.

As used herein, a "neutralizing antibody" is any antibody or antigen-binding fragment thereof that binds to a pathogen and interferes with the ability of the pathogen to infect a cell and/or cause disease in a subject.

"Peptide" as used herein includes peptides which are conservative variations of those peptides specifically exemplified herein. "Conservative variations" and "Conservative amino acid substitutions" as used herein denotes the replacement of an amino acid residue by another, biologically similar residue. Examples of conservative variations include, but are not limited to, the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic for aspartic acids, or glutamine for asparagine, and the like. Neutral hydrophilic amino acids which can be substituted for one another include asparagine, glutamine, serine and threonine.

"Conservative variations" also includes the use of a substituted amino acid in place of an unsubstituted parent amino acid provided that antibodies raised to the substituted polypeptide also immunoreact with the unsubstituted polypeptide. Such conservative substitutions are within the definition of the classes of the peptides of the invention. The biological activity of the peptides can be determined by standard methods known to those of skill in the art and described herein.

For nucleic acids, the term "substantial homology" indicates that two nucleic acids, or designated sequences thereof, when optimally aligned and compared, are identical, with appropriate nucleotide insertions or deletions, in at least about 80% of the nucleotides, at least about 90% to 95%, or at least about 98% to 99.5% of the nucleotides. Alternatively, substantial homology exists when the segments will hybridize under selective hybridization conditions, to the complement of the strand.

For polypeptides, the term "substantial homology" indicates that two polypeptides, or designated sequences thereof, when optimally aligned and compared, are identical, with appropriate amino acid insertions or deletions, in at least about 80% of the amino acids, at least about 90% to 95%, or at least about 98% to 99.5% of the amino acids.

The percent identity between two sequences is a function of the number of identical positions shared by the sequences (i.e., % homology = # of identical positions/total # of positions times 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The nucleic acids can be present in whole cells, in a cell lysate, or in a partially purified or substantially pure form. A nucleic acid is "isolated" or "rendered substantially pure" when purified away from other cellular components or other contaminants, e.g., other cellular nucleic acids (e.g., the other parts of the chromosome) or proteins, by standard techniques, including alkaline/SDS treatment, CsCI banding, column chromatography, agarose gel electrophoresis and others well known in the art. See, F. Ausubel, et al., ed. Current Protocols in Molecular Biology, Greene Publishing and Wiley Interscience, New York (1987).

Nucleic acids, e.g., cDNA, can be mutated, in accordance with standard techniques to provide gene sequences. For coding sequences, these mutations, can affect amino acid sequence as desired. In particular, DNA sequences substantially homologous to or derived from native V, D, J, constant, switches and other such sequences described herein are contemplated (where "derived" indicates that a sequence is identical or modified from another sequence).

The term "vector," as used herein, is intended to refer to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA or RNA segments can be ligated into the viral genome. Certain vectors are capable of autonomous replication in a host cell into which they are introduced (e.g., bacterial vectors having a bacterial origin of replication and episomal mammalian vectors).

Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors") In general, expression vectors of utility in recombinant DNA techniques are often in the form of plasmids. In the present specification, "plasmid" and "vector" can be used interchangeably as the plasmid is the most commonly used form of vector. However, also included are other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell that comprises a nucleic acid that is not naturally present in the cell, and can be a cell into which a recombinant expression vector has been introduced. It should be understood that such terms are intended to refer not only to the particular subject cell but to the progeny of such a cell. Because certain modifications can occur in succeeding generations due to either mutation or environmental influences, such progeny cannot, in fact, be identical to the parent cell, but are still included within the scope of the term "host cell" as used herein.

As used herein, the term "linked" refers to the association of two or more molecules. The linkage can be covalent or non-covalent. The linkage also can be genetic (i.e., recombinantly fused). Such linkages can be achieved using a wide variety of art recognized techniques, such as chemical conjugation and recombinant protein production.

An "effector function" refers to the interaction of an antibody Fc region with an Fc receptor or ligand, or a biochemical event that results therefrom. Exemplary "effector functions" include C1q binding, complement dependent cytotoxicity (CDC), Fc receptor binding, FcγR-mediated effector functions such as ADCC and antibody dependent cell-mediated phagocytosis (ADCP), and downregulation of a cell surface receptor (e.g., the B cell receptor; BCR). Such effector functions generally require the Fc region to be combined with a binding domain (e.g., an antibody variable domain).

An "Fc receptor" or "FcR" is a receptor that binds to the Fc region of an immunoglobulin. FcRs that bind to an IgG antibody comprise receptors of the FcγR family, including allelic variants and alternatively spliced forms of these receptors. The FcγR family consists of three activating (FcγRI, FcγRIII, and Fc.RIV in mice; FcγRIA, FcγRIIA, and FcγRIIIA in humans) and one inhibitory (FcγRIIB) receptor. Various properties of human FcγRs are known in the art. The majority of innate effector cell types coexpress one or more activating FcγR and the inhibitory FcγRIIB, whereas natural killer (NK) cells selectively express one activating Fc receptor (FcγRIII in mice and FcγRIIIA in humans) but not the inhibitory FcγRIIB in mice and humans. Human IgG1 binds to most human Fc receptors and is considered equivalent to murine IgG2a with respect to the types of activating Fc receptors that it binds to.

"Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (C1q) of the classical complement system. Thus, an Fc region comprises the constant region of an antibody excluding the first constant region immunoglobulin domain (e.g., CH1 or CL).

A "native sequence Fc region" or "native sequence Fc" comprises an amino acid sequence that is identical to the amino acid sequence of an Fc region found in nature. Native sequence human Fc regions include a native sequence human IgG1 Fc region; native sequence human IgG2 Fc region; native sequence human IgG3 Fc region; and native sequence human IgG4 Fc region as well as naturally occurring variants thereof. Native sequence Fc include the various allotypes of Fcs (see, e.g., Jefferis et al., mAbs 1: 1 (2009)).

A "variant sequence Fc region" or "non-naturally occurring Fc" comprises a modification, typically to alter one or more of its functional properties, such as serum half-life, complement fixation, Fc-receptor binding, protein stability and/or antigen-dependent cellular cytotoxicity, or lack thereof, among others. In some embodiments, the antibodies of the present disclosure can be chemically modified (e.g., one or more chemical moieties can be attached to the antibody) or be modified to alter its glycosylation, again to alter one or more functional properties of the antibody.

The terms "hinge," "hinge domain," "hinge region," and "antibody hinge region" refer to the domain of a heavy chain constant region that joins the CH1 domain to the CH2 domain and includes the upper, middle, and lower portions of the hinge (Roux et al., J Immunol 161:4083 (1998)). The hinge provides varying levels of flexibility between the binding and effector regions of an antibody and also provides sites for intermolecular disulfide bonding between the two heavy chain constant regions. The sequence of wildtype IgG1, IgG₂, IgG3, and IgG4 hinges are known in the art (e.g., International PCT publication no. WO 2017/087678). In one embodiment, the hinge region of CH1 of the antibodies is modified such that the number of cysteine residues in the hinge region is altered, e.g., increased or decreased. This approach is described further for instance in U.S. Pat. No. 5,677,425.

The constant region may be modified to stabilize the antibody, e.g., to reduce the risk of a bivalent antibody separating into two monovalent VH-VL fragments. For example, in an IgG4 constant region, residue S228 (residue numbering according to the EU index) may be mutated to a proline (P) residue to stabilize inter heavy chain disulphide bridge formation at the hinge (see, e.g., Angal et al., Mol Immunol. 30: 105-8(1995)). Antibodies or fragments thereof can also be defined in terms of their complementarity-determining regions (CDRs).

The term "complementarity-determining region" or "hypervariable region", when used herein, refers to the regions of an antibody in which amino acid residues involved in antigen binding are situated. The region of hypervariability or CDRs can be identified as the regions with the highest variability in amino acid alignments of antibody variable domains. Databases can be used for CDR identification such as the Kabat database, the CDRs e.g., being defined as comprising amino acid residues 24-34 (CDR1), 50-59 (CDR2) and 89-97 (CDR3) of the light-chain variable domain, and 31-35 (CDR1), 50-65 (CDR2) and 95-102 (CDR3) in the heavy-chain variable domain; (Kabat et al. 1991; Sequences of Proteins of Immunological Interest, Fifth Edition, U.S. Department of Health and Human Services, NIH Publication No. 91-3242). Alternatively, CDRs can be defined as those residues from a "hypervariable loop" (residues 26-33 (L1), 50-52 (L2) and 91-96 (L3) in the light-chain variable domain and 26-32 (H1), 53-55 (H2) and 96-101 (H3) in the heavy-chain variable domain (Chothia and Lesk, J. Mol. Biol 196: 901-917 (1987)). The CDR regions of the antibody sequences described herein are preferably defined according to the numbering scheme of IMGT which is an adaptation of the numbering scheme of Chothia (ImMunoGeneTics information system^{®}; Lefranc, M.-P. et al. IMGT, the international ImMunoGeneTics database. Nucleic Acids Res 27, 209-212 (1999).; http://imgt.org).

The term "epitope" or "antigenic determinant" refers to a site on an antigen to which an immunoglobulin or antibody specifically binds, e.g., as defined by the specific method used to identify it. Epitopes can be formed both from contiguous amino acids (usually a linear epitope) or noncontiguous amino acids juxtaposed by tertiary folding of a protein (usually a conformational epitope). Epitopes formed from contiguous amino acids are typically, but not always, retained on exposure to denaturing solvents, whereas epitopes formed by tertiary folding are typically lost on treatment with denaturing solvents.

An epitope typically includes at least 3, 4, 5, 6, 7, 8, 9, 10, 11, 12, 13, 14 or 15 amino acids in a unique spatial conformation. Methods for determining what epitopes are bound by a given antibody (i.e., epitope mapping) are well known in the art and include, for example, immunoblotting and immunoprecipitation assays, wherein overlapping or contiguous peptides from (e.g., from the spike (S) protein of SARS-CoV-2) are tested for reactivity with a given antibody. Methods of determining spatial conformation of epitopes include techniques in the art, for example, x-ray crystallography, antigen mutational analysis, 2-dimensional nuclear magnetic resonance and HDX-MS (see, e.g., Epitope Mapping Protocols in Methods in Molecular Biology, Vol. 66, G. E. Morris, Ed. (1996)).

The term "binds to the same epitope" with reference to two or more antibodies means that the antibodies bind to the same segment of amino acid residues, as determined by a given method. Techniques for determining whether antibodies bind to the "same epitope " with the antibodies described herein include, for example, epitope mapping methods, such as, x-ray analyses of crystals of antigen:antibody complexes which provides atomic resolution of the epitope and hydrogen/deuterium exchange mass spectrometry (HDX-MS).

Other methods monitor the binding of the antibody to antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same V_{H} and V_{L} or the same CDR1, 2 and 3 sequences are expected to bind to the same epitope.

Antibodies that "compete with another antibody for binding to a target" refer to antibodies that inhibit (partially or completely) the binding of the other antibody to the target. Whether two antibodies compete with each other for binding to a target, i.e., whether and to what extent one antibody inhibits the binding of the other antibody to a target, can be determined using known competition experiments, e.g., BIACORE^{™} surface plasmon resonance (SPR) analysis. In certain embodiments, an antibody competes with, and inhibits binding of another antibody to a target by at least 50%, 60%, 70%, 80%, 90% or 100%. The level of inhibition or competition can be different depending on which antibody is the "blocking antibody" (i.e., the cold antibody that is incubated first with the target).

Competition assays can be conducted as described, for example, in Ed Harlow and David Lane, Cold Spring Harb Protoc; 2006; doi: 10.1101/pdb.prot4277 or in Chapter 11 of "Using Antibodies" by Ed Harlow and David Lane, Cold Spring Harbor Laboratory Press, Cold Spring Harbor, N.Y., USA 1999. Two antibodies "cross-compete" if antibodies block each other both ways by at least 50%, i.e., regardless of whether one or the other antibody is contacted first with the antigen in the competition experiment.

As used herein, the terms "specific binding," "selective binding," "selectively binds," and "specifically binds," refer to antibody binding to an epitope on a predetermined antigen. Preferably, the antibody binds to the predetermined antigen with an affinity that is at least twofold greater than its affinity for binding to a non-specific antigen (e.g., BSA, casein) other than the predetermined antigen or a closely-related antigen.

The term "binding affinity" herein refers to a measurement of the strength of a non-covalent interaction between two molecules, e.g. an antibody, or fragment thereof, and an antigen. The term "binding affinity" is used to describe monovalent interactions (intrinsic activity).

The binding affinity between two molecules, e.g. an antibody, or fragment thereof, and an antigen, through a monovalent interaction may be quantified by determination of the equilibrium dissociation constant (K_{D}). In turn, K_{D} can be determined by measurement of the kinetics of complex formation and dissociation, e.g. by the SPR method. The rate constants corresponding to the association and the dissociation of a monovalent complex are referred to as the association rate constant kₐ (or kₒₙ) and dissociation rate constant k_{d} (or k_{off}), respectively. K_{D} is related to kₐ and k_{d} through the equation K_{D}=k_{d}/kₐ. Following the above definition, binding affinities associated with different molecular interactions, such as comparison of the binding affinity of different antibodies for a given antigen, may be compared by comparison of the K_{D} values for the individual antibody/antigen complexes.

The term "binding specificity" herein refers to the interaction of a molecule such as an antibody, or fragment thereof, with a single exclusive antigen, or with a limited number of highly homologous antigens (or epitopes). In contrast, antibodies that are capable of specifically binding to the spike (S) protein of SARS-CoV-2 are not capable of binding dissimilar molecules.

The specificity of an interaction and the value of an equilibrium binding constant can be determined directly by well-known methods. Standard assays to evaluate the ability of ligands (such as antibodies) to bind their targets are known in the art and include, for example, ELISAs, Western blots, RIAs, and flow cytometry analysis. The binding kinetics and binding affinity of the antibody also can be assessed by standard assays known in the art, such as SPR.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

A "polypeptide" refers to a chain comprising at least two consecutively linked amino acid residues, with no upper limit on the length of the chain. One or more amino acid residues in the protein can contain a modification such as, but not limited to, glycosylation, phosphorylation or disulfide bond formation. A "protein" can comprise one or more polypeptides.

The term "nucleic acid molecule," as used herein, is intended to include DNA molecules and RNA molecules. A nucleic acid molecule can be single-stranded or double-stranded, and can be cDNA.

The term "subject" includes human and other mammalian subjects that receive either prophylactic or therapeutic treatment. As used herein, the term "subject" includes any human or non-human animal. The term "non-human animal" includes all vertebrates, e.g., mammals and non-mammals, such as non-human primates, sheep, dog, cow, chickens, amphibians, reptiles, etc.

As used herein, the terms "ug" and "uM" are used interchangeably with "µg" and "µM," respectively.

As used herein, "administering" refers to the physical introduction of a composition comprising a therapeutic agent to a subject, using any of the various methods and delivery systems known to those skilled in the art. Different routes of administration for the antibodies described herein include intravenous, intraperitoneal, intramuscular, subcutaneous, spinal or other parenteral routes of administration, for example by injection or infusion.

The phrase "parenteral administration" as used herein means modes of administration other than enteral and topical administration, usually by injection, and includes, without limitation, intravenous, intraperitoneal, intramuscular, intraarterial, intrathecal, intralymphatic, intralesional, intracapsular, intraorbital, intracardiac, intradermal, transtracheal, subcutaneous, subcuticular, intraarticular, subcapsular, subarachnoid, intraspinal, epidural and intrasternal injection and infusion, as well as in vivo electroporation.

Alternatively, an antibody described herein can be administered via a non-parenteral route, such as a topical, epidermal or mucosal route of administration, for example, intranasally, orally, vaginally, rectally, sublingually or topically. Administering can also be performed, for example, once, a plurality of times, and/or over one or more extended periods.

As used herein "vaccination composition" means a pharmaceutical compostion comprising at least one antibody or antigen-binding portion thereof of the present invention which is capable of providing active and/or passive immunity. "Active immunity" as used herein means inducing or enhancing a subject's immune response to an antigen. "Passive immunity" as used herein means supplementing a subject's immune response to an antigen or pathogen by providing antibodies and/or antigen-binding portions thereof which neutralize an antigen.

The present inventors have dedicated themselves to solving the problem of the present invention and were successful to find novel and useful ultrapotent human monoclonal antibodies against SARS-related coronavirus having superior neutralization potency against circulating SARS-CoV-2 variants of concern as well as emerging escape variants in comparison to publicly available neutralizing antibodies.

The inventors recently demonstrated that a small fraction of COVID-19 convalescent individuals mount a highly potent antibody response to SARS-CoV-2 with a high degree of cross-reactivity to SARS-CoV-1 (Vanshylla, K. et al. (2021). Cell Host Microbe 29, 917-929). In these elite neutralizers, the antibody response was analyzed in detail at a single B-cell level, including the isolation and characterization of 126 monoclonal antibodies from 10 individuals.

Sequence analysis of 1,361 SARS-CoV-2-reactive B cells divulged a largely polyclonal but convergent antibody response. Several antibodies were sarbecovirus cross-reactive, with some displaying merbecovirus- and embecovirus-reactivity. Based on a comprehensive neutralization map, several broadly neutralizing antibodies (bNAbs) could be identified as part of the present invention that were highly effective against the circulating Wu-01, B.1, B.1.1.7, B1.351, B.1.429, B.1.617, and B.1.617.2 SARS-CoV-2 variants as well as various prominent sites of antigenic escape.

In addition, the inventors were able to identify new sites of possible antigenic escape based on a timed phylogenetic tree of circulating global SARS-CoV-2 variants using over 716,806 unique quality-controlled sequences (data not shown). Using this global data, four RBD mutants were identified as potential candidates as emerging spike escape sites, R346S, Q414H, N440K, and T478K.

While these mutants are not currently circulating at high frequency, they may be playing a role in the future and have thus been included in the analysis of neutralization potency of the antibodies of the present invention. Strikingly, the antibodies of the present invention were also able to neutralize these RBD mutants with high potency, in contrast to clinically tested antibodies such as DZIF-10c, S309, or REGN10987.

Therefore, these bNAbs isolated from elite neutralizers, serve as an important resource to combat currently circulating and emerging SARS-CoV-2 variants as well as potential future CoV pandemics caused by such viruses.

Accordingly, the present invention provides antibodies or antigen-binding fragments thereof directed against SARS-related coronavirus, wherein the antibody or antigen-binding fragment thereof comprises the combination of the heavy chain CDR1 to CDR3 and the light chain CDR1 to CDR3 amino acid sequence of one antibody selected from the group comprising:
R200-1B9 (having a CDR-H1 amino acid sequence of SEQ ID No. 27, a CDR-H2 amino acid sequence of SEQ ID No. 28, a CDR-H3 amino acid sequence of SEQ ID No. 29, a CDR-L1 amino acid sequence of SEQ ID No. 30, a CDR-L2 amino acid sequence of SEQ ID No. 31, a CDR-L3 amino acid sequence of SEQ ID No. 32),
R207-2F11 (having a CDR-H1 amino acid sequence of SEQ ID No. 33, a CDR-H2 amino acid sequence of SEQ ID No. 34, a CDR-H3 amino acid sequence of SEQ ID No. 35, a CDR-L1 amino acid sequence of SEQ ID No. 36, a CDR-L2 amino acid sequence of SEQ ID No. 37, a CDR-L3 amino acid sequence of SEQ ID No. 38),
R40-1G8 (having a CDR-H1 amino acid sequence of SEQ ID No. 39, a CDR-H2 amino acid sequence of SEQ ID No. 40, a CDR-H3 amino acid sequence of SEQ ID No. 41, a CDR-L1 amino acid sequence of SEQ ID No. 42, a CDR-L2 amino acid sequence of SEQ ID No. 43, a CDR-L3 amino acid sequence of SEQ ID No. 44),
R568-2G5 (having a CDR-H1 amino acid sequence of SEQ ID No. 45, a CDR-H2 amino acid sequence of SEQ ID No. 46, a CDR-H3 amino acid sequence of SEQ ID No. 47, a CDR-L1 amino acid sequence of SEQ ID No. 48, a CDR-L2 amino acid sequence of SEQ ID No. 49, a CDR-L3 amino acid sequence of SEQ ID No. 50),
R568-2B11 (having a CDR-H1 amino acid sequence of SEQ ID No. 51, a CDR-H2 amino acid sequence of SEQ ID No. 52, a CDR-H3 amino acid sequence of SEQ ID No. 53, a CDR-L1 amino acid sequence of SEQ ID No. 54, a CDR-L2 amino acid sequence of SEQ ID No. 55, a CDR-L3 amino acid sequence of SEQ ID No. 56),
R207-2G4 (having a CDR-H1 amino acid sequence of SEQ ID No. 57, a CDR-H2 amino acid sequence of SEQ ID No. 58, a CDR-H3 amino acid sequence of SEQ ID No. 59, a CDR-L1 amino acid sequence of SEQ ID No. 60, a CDR-L2 amino acid sequence of SEQ ID No. 61, a CDR-L3 amino acid sequence of SEQ ID No. 62),
R40-1C8 (having a CDR-H1 amino acid sequence of SEQ ID No. 63, a CDR-H2 amino acid sequence of SEQ ID No. 64, a CDR-H3 amino acid sequence of SEQ ID No. 65, a CDR-L1 amino acid sequence of SEQ ID No. 66, a CDR-L2 amino acid sequence of SEQ ID No. 67, a CDR-L3 amino acid sequence of SEQ ID No. 68),
R568-2B9 (having a CDR-H1 amino acid sequence of SEQ ID No. 69, a CDR-H2 amino acid sequence of SEQ ID No. 70, a CDR-H3 amino acid sequence of SEQ ID No. 71, a CDR-L1 amino acid sequence of SEQ ID No. 72, a CDR-L2 amino acid sequence of SEQ ID No. 73, a CDR-L3 amino acid sequence of SEQ ID No. 74),
R568-1B3 (having a CDR-H1 amino acid sequence of SEQ ID No. 75, a CDR-H2 amino acid sequence of SEQ ID No. 76, a CDR-H3 amino acid sequence of SEQ ID No. 77, a CDR-L1 amino acid sequence of SEQ ID No. 78, a CDR-L2 amino acid sequence of SEQ ID No. 79, a CDR-L3 amino acid sequence of SEQ ID No. 80),
R568-2E1 (having a CDR-H1 amino acid sequence of SEQ ID No. 81, a CDR-H2 amino acid sequence of SEQ ID No. 82, a CDR-H3 amino acid sequence of SEQ ID No. 83, a CDR-L1 amino acid sequence of SEQ ID No. 84, a CDR-L2 amino acid sequence of SEQ ID No. 85, a CDR-L3 amino acid sequence of SEQ ID No. 86),
R568-1G9 (having a CDR-H1 amino acid sequence of SEQ ID No. 87, a CDR-H2 amino acid sequence of SEQ ID No. 88, a CDR-H3 amino acid sequence of SEQ ID No. 89, a CDR-L1 amino acid sequence of SEQ ID No. 90, a CDR-L2 amino acid sequence of SEQ ID No. 91, a CDR-L3 amino acid sequence of SEQ ID No. 92),
R121-1F1 (having a CDR-H1 amino acid sequence of SEQ ID No. 93, a CDR-H2 amino acid sequence of SEQ ID No. 94, a CDR-H3 amino acid sequence of SEQ ID No. 95, a CDR-L1 amino acid sequence of SEQ ID No. 96, a CDR-L2 amino acid sequence of SEQ ID No. 97, a CDR-L3 amino acid sequence of SEQ ID No. 98),
R259-1B9 (having a CDR-H1 amino acid sequence of SEQ ID No. 99, a CDR-H2 amino acid sequence of SEQ ID No. 100, a CDR-H3 amino acid sequence of SEQ ID No. 101, a CDR-L1 amino acid sequence of SEQ ID No. 102, a CDR-L2 amino acid sequence of SEQ ID No. 103, a CDR-L3 amino acid sequence of SEQ ID No. 104).

Within the context of the present invention, the antibodies, which have been generated and described herein, may be used and claimed as the complete monoclonal human antibody or as any functional or antigen-binding fragment thereof. Preferably, the antibody or any kind of functional or antigen-binding fragment thereof should at least comprise the complementarity determining regions (CDR) 1 to 3 of the heavy chain and CDR 1 to 3 of the light chain of the antibody.

The CDR regions of the antibody sequences described herein are preferably defined according to the numbering scheme of IMGT which is an adaptation of the numbering scheme of Chothia (ImMunoGeneTics information system^{®}; Lefranc, M.-P. et al. IMGT, the international ImMunoGeneTics database. Nucleic Acids Res 27, 209-212 (1999); http://imgt.org).

Based on the common general knowledge and the information given herein on the variable region heavy chain amino acid sequences and the variable region light chain amino acid sequences of the antibodies of the invention, the CDRs can be easily and unambiguously determined by a skilled person.

According to one preferred embodiment of the present invention, the CDR sequences of the light and heavy chain sequences of the antibodies and antigen-binding fragments thereof described herein are as follows:

| **SEQ ID NO of V domains:** | **Source** | **CDR1** | **SEQ ID of CDR1** | **CDR2** | **SEQ ID of CDR2** | **CDR3** | **SEQ ID of CDR3** |
|---|---|---|---|---|---|---|---|
| 1 | Heavy chain V domain of R200-1B9 | GFTFDSSA | 27 | IVVGSGN T | 28 | AAPHCNRTT CYDAFDL | 29 |
| 2 | Light chain V domain of R200-1B9 | QSVRSSY | 30 | GPS | 31 | QQYDRSPW T | 32 |
| 3 | Heavy chain V domain of R207-2F11 | GLIVSSNY | 33 | IYPGGTT | 34 | ARDLVYRGM DV | 35 |
| 4 | Light chain V domain of R207-2F11 | QDINKY | 36 | DAS | 37 | | 38 |
| 5 | Heavy chain V domain of R40-1G8 | GLTVSSNY | 39 | IYAGGST | 40 | ARDLYVFGM DV | 41 |
| 6 | Light chain V domain of R40-1G8 | QGISSY | 42 | AAS | 43 | | 44 |
| 7 | Heavy chain V domain of R568-2G5 | GVTVSSNY | 45 | IYSGGST | 46 | ARDLYYYGM DV | 47 |
| 8 | Light chain V domain of R568-2G5 | QGIGNY | 48 | AAS | 49 | QQVNSYPPE CT | 50 |
| 9 | Heavy chain V domain of R568-2B11 | GLIVSSNY | 51 | IYAGGST | 52 | TRDLVYYGM DV | 53 |
| 10 | Light chain V domain of R568-2B11 | QGISNY | 54 | AAS | 55 | QQLNSYPPD T | 56 |
| 11 | Heavy chain V domain of R207-2G4 | GLIVSSNY | 57 | LYSGGST | 58 | ARDLVAYGM DV | 59 |
| 12 | Light chain V domain of R207-2G4 | QGISSH | 60 | AAS | 61 | QHLEM | 62 |
| 13 | Heavy chain V domain of R40-1C8 | GVTVSRNY | 63 | IYAGGST | 64 | VRDLVDYGM DV | 65 |
| 14 | Light chain V domain of R40-1C8 | QGINSY | 66 | GAS | 67 | QQLNSYSRD T | 68 |
| 15 | Heavy chain V domain of R568-2B9 | GITVSSNY | 69 | MYAGGST | 70 | ARDLVHYGM DV | 71 |
| 16 | Light chain V domain of R568-2B9 | QGISSY | 72 | AAS | 73 | QQLTNYPPD T | 74 |
| 17 | Heavy chain V domain of R568-1B3 | GLIVSSNY | 75 | LYAGGSS | 76 | ARDLVAYGM DV | 77 |
| 18 | Light chain V domain of R568-1B3 | QGISSS | 78 | GAS | 79 | QQLNSDLYT | 80 |
| 19 | Heavy chain V domain of R568-2E1 | GLTVSSNY | 81 | IYSGGTT | 82 | ARDLIVYGM DV | 83 |
| 20 | Light chain V domain of R568-2E1 | QGISNY | 84 | GAS | 85 | QQLNSDFFT | 86 |
| 21 | Heavy chain V domain of R568-1G9 | GITVSSNY | 87 | IYSGGST | 88 | ARDLVVLYG MDV | 89 |
| 22 | Light chain V domain of R568-1G9 | SSNIGAGYD | 90 | GNS | 91 | QSYDSSLSAL | 92 |
| 23 | Heavy chain V domain of R121-1F1 | GFTFSRDG | 93 | ISYDGSNK | 94 | AKDGPQAFG TYFDY | 95 |
| 24 | Light chain V domain of R121-1F1 | SSNIGSGYV | 96 | GNN | 97 | QSYDSRLSGV V | 98 |
| 25 | Heavy chain V domain of R259-1B9 | GFTFSSSA | 99 | IVVGSGN T | 100 | AAPYCTGGS CFDAFDI | 101 |
| 26 | Light chain V domain of R259-1B9 | QSVRSSY | 102 | GPS | 103 | QQYGSSPWT | 104 |

According to one embodiment of the present invention, the antibody or antigen-binding fragment thereof comprises a heavy chain amino acid sequence of antibody R200-1B9 (SEQ ID No. 121), or a heavy chain amino acid sequence of antibody R207-2F11 (SEQ ID No. 123), or a heavy chain amino acid sequence of antibody R40-1G8 (SEQ ID No. 125), or a heavy chain amino acid sequence of antibody R568-2G5 (SEQ ID No. 127), or a heavy chain amino acid sequence of antibody R568-2B11 (SEQ ID No. 129), or a heavy chain amino acid sequence of antibody R207-2G4 (SEQ ID No. 131), or a heavy chain amino acid sequence of antibody R40-1C8 (SEQ ID No. 133), or a heavy chain amino acid sequence of antibody R568-2B9 (SEQ ID No. 135), or a heavy chain amino acid sequence of antibody R568-1B3 (SEQ ID No. 137), or a heavy chain amino acid sequence of antibody R568-2E1 (SEQ ID No. 139), or a heavy chain amino acid sequence of antibody R568-1G9 (SEQ ID No. 141), or a heavy chain amino acid sequence of antibody R121-1F1 (SEQ ID No. 143), or a heavy chain amino acid sequence of antibody R259-1B9 (SEQ ID No. 145).

According to an embodiment of the present invention, the antibody or antigen-binding fragment thereof comprises a light chain amino acid sequence of antibody R200-1B9 (SEQ ID No. 122), or a light chain amino acid sequence of antibody R207-2F11 (SEQ ID No. 124), or a light chain amino acid sequence of antibody R40-1G8 (SEQ ID No. 126), or a light chain amino acid sequence of antibody R568-2G5 (SEQ ID No. 128), or a light chain amino acid sequence of antibody R568-2B11 (SEQ ID No. 130), or a light chain amino acid sequence of antibody R207-2G4 (SEQ ID No. 132), or a light chain amino acid sequence of antibody R40-1C8 (SEQ ID No. 134), or a light chain amino acid sequence of antibody R568-2B9 (SEQ ID No. 136), or a light chain amino acid sequence of antibody R568-1B3 (SEQ ID No. 138), or a light chain amino acid sequence of antibody R568-2E1 (SEQ ID No. 140), or a light chain amino acid sequence of antibody R568-1G9 (SEQ ID No. 142), or a light chain amino acid sequence of antibody R121-1F1 (SEQ ID No. 144), or a light chain amino acid sequence of antibody R259-1B9 (SEQ ID No. 146).

According to a preferred embodiment of the present invention, the antibody comprises a heavy chain amino acid sequence of SEQ ID No. 121 and a light chain amino acid sequence of SEQ ID No. 122, or the antibody comprises a heavy chain amino acid sequence of SEQ ID No. 123 and a light chain amino acid sequence of SEQ ID No. 124, or the antibody comprises a heavy chain amino acid sequence of SEQ ID No. 125 and a light chain amino acid sequence of SEQ ID No. 126, or the antibody comprises a heavy chain amino acid sequence of SEQ ID No. 127 and a light chain amino acid sequence of SEQ ID No. 128, or the antibody comprises a heavy chain amino acid sequence of SEQ ID No. 129 and a light chain amino acid sequence of SEQ ID No. 130, or the antibody comprises a heavy chain amino acid sequence of SEQ ID No. 131 and a light chain amino acid sequence of SEQ ID No. 132, or the antibody comprises a heavy chain amino acid sequence of SEQ ID No. 133 and a light chain amino acid sequence of SEQ ID No. 134, or the antibody comprises a heavy chain amino acid sequence of SEQ ID No. 135 and a light chain amino acid sequence of SEQ ID No. 136, or the antibody comprises a heavy chain amino acid sequence of SEQ ID No. 137 and a light chain amino acid sequence of SEQ ID No. 138, or the antibody comprises a heavy chain amino acid sequence of SEQ ID No. 139 and a light chain amino acid sequence of SEQ ID No. 140, or the antibody comprises a heavy chain amino acid sequence of SEQ ID No. 141 and a light chain amino acid sequence of SEQ ID No. 142, or the antibody comprises a heavy chain amino acid sequence of SEQ ID No. 143 and a light chain amino acid sequence of SEQ ID No. 144, or the antibody comprises a heavy chain amino acid sequence of SEQ ID No. 145 and a light chain amino acid sequence of SEQ ID No. 146.

According to a specific embodiment of the present invention, the antibody consists of two heavy chains of sequence SEQ ID NO: 121 and two light chains of sequence SEQ ID NO: 122, or the antibody consists of two heavy chains of sequence SEQ ID NO: 123 and two light chains of sequence SEQ ID NO: 124, or the antibody consists of two heavy chains of sequence SEQ ID NO: 125 and two light chains of sequence SEQ ID NO: 126, or the antibody consists of two heavy chains of sequence SEQ ID NO: 127 and two light chains of sequence SEQ ID NO: 128, or the antibody consists of two heavy chains of sequence SEQ ID NO: 129 and two light chains of sequence SEQ ID NO: 130, or the antibody consists of two heavy chains of sequence SEQ ID NO: 131 and two light chains of sequence SEQ ID NO: 132, or the antibody consists of two heavy chains of sequence SEQ ID NO: 133 and two light chains of sequence SEQ ID NO: 134, or the antibody consists of two heavy chains of sequence SEQ ID NO: 135 and two light chains of sequence SEQ ID NO: 136, or the antibody consists of two heavy chains of sequence SEQ ID NO: 137 and two light chains of sequence SEQ ID NO: 138, or the antibody consists of two heavy chains of sequence SEQ ID NO: 139 and two light chains of sequence SEQ ID NO: 140, or the antibody consists of two heavy chains of sequence SEQ ID NO: 141 and two light chains of sequence SEQ ID NO: 142, or the antibody consists of two heavy chains of sequence SEQ ID NO: 143 and two light chains of sequence SEQ ID NO: 144, or the antibody consists of two heavy chains of sequence SEQ ID NO: 145 and two light chains of sequence SEQ ID NO: 146.

According to a preferred embodiment of the present invention, the antibody used as a source for sequences comprised in the antibody or antigen-binding fragment thereof according to the present invention is selected from the group comprising R200-1B9, R207-2F11, R40-1G8, R568-2G5, R568-2B11, R207-2G4, R40-1C8, R568-2B9, R568-1B3, R568-2E1, R568-1G9, R121-1F1, and R259-1B9, preferably from the group comprising R200-1B9, R207-2F11, R40-1G8, R568-2G5, R568-2B11, R207-2G4, R40-1C8, R568-2B9, and R568-1B3, more preferably from the group comprising R200-1B9, R207-2F11, R40-1G8, and R568-2G5.

In one embodiment of the present invention, the antibody used as a source for sequences comprised in the antibody of the invention is R200-1B9. In another embodiment of the present invention, the antibody used as a source for sequences comprised in the antibody of the invention is R207-2F11. In another embodiment of the present invention, the antibody used as a source for sequences comprised in the antibody of the invention is R40-1G8. In another embodiment of the present invention, the antibody used as a source for sequences comprised in the antibody of the invention is R568-2G5.

In another embodiment of the present invention, the antibody used as a source for sequences comprised in the antibody of the invention is R121-3G2. This antibody is able to efficiently cross-neutralize the SARS-related coronavirus strains SARS-CoV-1 and the Bat coronavirus WIV-1 in addition to different variants of SARS-CoV-2. In yet another embodiment of the present invention, the antibody used as a source for sequences comprised in the antibody of the invention is R40-1E1. This antibody is able to also efficiently cross-neutralize the SARS-related coronavirus strains SARS-CoV-1 and the Bat coronavirus WIV-1 in addition to different variants of SARS-CoV-2.

According to another preferred embodiment of the present invention, the SARS-related coronavirus strain is severe acute respiratory syndrome coronavirus 2 (SARS-CoV-2).

According to one embodiment of the present invention, the amino acid sequences of the CDRs or of the variable regions comprised in the antibody or antigen-binding fragment thereof according to the present invention are from an antibody which is able to neutralize each of the SARS-CoV-2 lineages B.1, B.1.1.7, B.1.351, B.1.429, B.1.617 and B.1.617.2 in a pseudovirus neutralization assay as described in the description with an IC50 of at most 0.4 µg/ml, preferably at most 0.1 µg/ml, more preferably at most 0.05 µg/ml, even more preferably at most 0.025 µg/ml, even more preferably at most 0.01 µg/ml, particularly preferably at most 0.0025 µg/ml.

According to another embodiment of the present invention, the amino acid sequences of the CDRs or of the variable regions comprised in the antibody or antigen-binding fragment thereof according to the present invention are from an antibody which is able to neutralize each of the SARS-CoV-2 spike escape mutants 69-70del, K417E, N439K, K444Q, V445A, G446V, L452R, Y453F, L455F, G476S, S477N, E484K, F486V, F490S, Q493R, Q493K, S494P, N501Y in a pseudovirus neutralization assay as described in the description with an IC50 of at most 1.3 µg/ml, preferably at most 0.5 µg/ml, more preferably at most 0.05 µg/ml, even more preferably at most 0.02 µg/ml, particularly preferably at most 0.01 µg/ml.

According to one embodiment of the present invention, the amino acid sequences of the CDRs or of the variable regions comprised in the antibody or antigen-binding fragment thereof are taken from an antibody which is able to neutralize each of the emerging SARS-CoV-2 escape variants R346S, Q414H, N440K, and T478K in a pseudovirus neutralization assay as described in the description with an IC50 of at most 0.0015 µg/ml.

According to an embodiment of the present invention, the amino acid sequences of the CDRs or of the variable regions comprised in the antibody or antigen-binding fragment thereof according to the present invention are from an antibody which is able to neutralize the SARS-CoV-2 variants Wu-01, B.1, B.1.1.7, B.1.351, B.1.429, B.1.617 and B.1.617.2 and the SARS-CoV-2 escape mutants 69-70del, K417E, N439K, K444Q, V445A, G446V, L452R, Y453F, L455F, G476S, S477N, E484K, F486V, F490S, Q493R, Q493K, S494P, N501Y, R346S, Q414H, N440K, and T478K in a pseudovirus neutralization assay as described in the description across all variants and mutants with an average IC50 of at most 0.08 µg/ml, preferably of at most 0.025 µg/ml, more preferably of at most 0.01 µg/ml, even more preferably of at most 0.006 µg/ml, particularly preferably of at most 0.002 µg/ml.

According to the present invention, the pseudovirus neutralization assay for determining IC₅₀ values is to be carried out as described in the section *"Pseudovirus neutralization assay to determine IgG neutralizing activity"* below.

According to one embodiment of the present invention, the antibody or antigen-binding fragment thereof according to the present invention does not display autoreactivity defined as detectable binding when tested against permeabilized HEp-2 cells using an antinuclear antibody (ANA) testing kit (NOVA-Lite HEp-2 ANA kit; Inova Diagnostics) at concentrations of 100 µg/ml of the antibody or antigen-binding fragment thereof.

According to a preferred embodiment of the present invention, the assay for determining autoreactivity is to be carried out as described in the section *"Hep-2 autoreactivity* assay" below.

According to an embodiment of the present invention, the CDR sequences of the light and heavy chain sequences of the antibodies described herein are as follows:

| **SEQ ID NO of V domains:** | **Source** | **CDR1** | **SEQ ID of CDR1** | **CDR2** | **SEQ ID of CDR2** | **CDR3** | **SEQ ID of CDR3** |
|---|---|---|---|---|---|---|---|
| 105 | Heavy chain V domain of R121-3G2 | GGSIASTYY Y | 109 | IHYNGST | 110 | ARYGPYYYD RSGSLLSDPF DN | 111 |
| 106 | Light chain V domain of R121-3G2 | QGLSSSS | 112 | GAS | 113 | QQYSNSPLT | 114 |
| 107 | Heavy chain V domain of R40-1E1 | GFTFSRYG | 115 | IWYDGNN K | 116 | ARALQYYDIL TGDYSVVRS DAFDI | 117 |
| 108 | Light chain V domain of R40-1E1 | QSVSGTY | 118 | GAS | 119 | QQYGSSPLT | 120 |

According to one embodiment of the present invention, the antibody has a heavy chain amino acid sequence of antibody R121-3G2 (SEQ ID No. 147), or a heavy chain amino acid sequence of antibody R40-1E1 (SEQ ID No. 149). According to an embodiment of the present invention, the antibody has a light chain amino acid sequence of antibody R121-3G2 (SEQ ID No. 148), or a light chain amino acid sequence of antibody R40-1E1 (SEQ ID No. 150).

According to a preferred embodiment of the present invention, the antibody comprises a heavy chain amino acid sequence of SEQ ID No. 147 and a light chain amino acid sequence of SEQ ID No. 148, or the antibody comprises a heavy chain amino acid sequence of SEQ ID No. 149 and a light chain amino acid sequence of SEQ ID No. 150.

According to a specific embodiment of the present invention, the antibody consists of two heavy chains of sequence SEQ ID NO: 147 and two light chains of sequence SEQ ID NO: 148, or the antibody consists of two heavy chains of sequence SEQ ID NO: 149 and two light chains of sequence SEQ ID NO: 150.

In general, the antibodies or antigen-binding fragments thereof as described herein further encompass antibody amino acid sequences being at least 80% identical to the sequences as defined above as long as they are still directed against the spike (S) protein of SARS-CoV-2 as in SEQ ID NO. 151, preferably as long as they are still directed against the receptor-binding domain (RBD) of the spike (S) protein of SARS-CoV-2 as in SEQ ID NO. 152.

According to one embodiment, the specific sequence of the spike (S) protein of SARS-CoV-2 of SEQ ID NO. 151 or the receptor-binding domain thereof of SEQ ID NO. 152 against which the antibodies or antigen-binding fragments thereof should be directed may additionally include one or more of the sequence variations 69-70del, R346S, Q414H, K417E, N439K, N440K, K444Q, V445A, G446V, L452R, Y453F, L455F, G476S, S477N, T478K, E484K, F486V, F490S, Q493R, Q493K, S494P and N501Y.

According to one other embodiment, the specific sequence of the spike (S) protein of SARS-CoV-2 of SEQ ID NO. 151 or the receptor-binding domain thereof of SEQ ID NO. 152 against which the antibodies or antigen-binding fragments thereof should be directed may be taken from one of the commonly known SARS-CoV-2 variants Wu01, B.1, B.1.1.7, B1.351, B.1.429, B.1.617, or B.1.617.2.

The sequence variations encompassed herein are meant to include sequences having trivial mutations, i.e. conservative mutations, of the antibody amino acid sequence which do not interfere with structural folds and the affinity of the antibody to the spike (S) protein. Preferably, the deviations in the amino acid sequence leading to an at least 80%, 85%, 90% or 95% overall identity to the individualized sequences explicitly disclosed herein are present exclusively outside the CDR regions of the antibodies according to the invention. In particular, the present invention encompasses antibody amino acid sequences having 1, 2, 3, 4, 5, or 6 mutations within the constant regions of the antibody.

The antibodies according to the present invention are preferably of human origin. Thus, at least the sequences outside the CDRs, such as framework and constant regions of the antibody, are preferably of human origin or can be attributed to human origin. Furthermore, the antibodies of the present invention are preferably monoclonal.

In one preferred embodiment, the antibody is a monoclonal antibody or a fragment thereof that retains binding specificity and ability to neutralize infectious pathogen. In one preferred embodiment, the antibody is an IgG1, IgG2, IgG3, or IgG4 antibody. For example, the antibody may be an antibody comprising an Fc domain of any human IgG isotype (e.g. IgG1, IgG2, IgG3, or IgG4).

Optionally, the antigen-binding compound consists of or comprises a Fab, Fab', Fab'-SH, F(ab)₂, Fv, a diabody, single-chain antibody fragment, or a multispecific antibody comprising multiple different antibody fragments.

Within the present invention, an antibody or antigen-binding fragment directed against the spike (S) protein of SARS-CoV-2 means an antibody binding to the spike (S) protein of SARS-CoV-2 with an at least 10-fold, more preferably at least 50-fold, particularly preferably at least 100-fold increased affinity compared to unrelated epitopes, proteins or protein regions.

It is a trivial task for a skilled person to determine if an antibody which exhibits a certain degree of identity is directed against the spike (S) protein of SARS-CoV-2 based on the above or the common general knowledge.

The determination of percent identity between two sequences is accomplished according to the present invention by using the mathematical algorithm of Karlin and Altschul (Proc. Natl. Acad. Sci. USA (1993) 90: 5873-5877). Such an algorithm is the basis of the BLASTN and BLASTP programs of Altschul et al. (J. Mol. Biol. (1990) 215: 403-410). BLAST nucleotide searches are performed with the BLASTN program. To obtain gapped alignments for comparative purposes, Gapped BLAST is utilized as described by Altschul et al. (Nucleic Acids Res. (1997) 25: 3389-3402). When utilizing BLAST and Gapped BLAST programs, the default parameters of the respective programs are used.

According to a preferred embodiment of the present invention, antibody amino acid sequences form part of the invention which consist of or comprise a nucleic acid sequence being at least 85% identical to the sequences defined above and disclosed herein, more preferably at least 90% identical, even more preferred at least 95% identical.

According to a preferred embodiment of the present invention, the SARS-related coronavirus strain is severe acute respiratory syndrome coronavirus 2 (SARS-CoV-2) which may alternatively be referred to as SARS-related coronavirus 2 in the art. According to another preferred embodiment of the present invention, the SARS-related coronavirus strains are severe acute respiratory syndrome coronavirus (SARS-CoV or SARS-CoV-1) or the Bat SARS-like coronavirus WIV-1, preferably the severe acute respiratory syndrome coronavirus (SARS-CoV or SARS-CoV-1).

According to another preferred embodiment of the invention, the antibody or antigen-binding fragment thereof is directed against the ectodomain of the spike (S) protein of SARS-CoV-2.

According to a more preferred embodiment of the present invention, the antibody or antigen-binding fragment thereof is directed against the Wu01 spike (S) homotrimer of SARS-CoV-2 as described in Hoffmann M. et al. (2020). Cell 181, 271-280 (EPI_ISL_406716. This virus isolate has been studied intensively and is best understood at the time of filing.

However, preferably, the antibody or antigen-binding fragment thereof should also be directed against equivalent sequences of other virus variants. According to one specific embodiment, the antibody or antigen-binding fragment thereof is directed against the receptor-binding domain (RBD) of the spike (S) protein of SARS-CoV-2 (SEQ ID NO. 152).

According to a preferred embodiment of the present invention, the antibody or antigen-binding fragment thereof does not display autoreactivity against human cells defined as a detectable binding pattern when tested against permeabilized HEp-2 cells using an antinuclear antibody (ANA) testing kit (NOVA-Lite HEp-2 ANA kit; Inova Diagnostics) at concentrations of 100 µg/ml of the antibody or antigen-binding fragment thereof. Alternatively preferably, other assays known in the art may be used to determine or exclude autoreactivity of antibodies or antigen-binding fragments thereof.

In the description of the present application, antibody designations may be used. It is pointed out that the antibodies consist of heavy and light chains which also form part of the present description. If reference is made to an antibody by its designation or to a SEQ ID NO., it should be understood that these ways of reference are interchangeable.

The present invention further relates to a pharmaceutical composition comprising an antibody or antigen-binding fragment thereof according to the invention as defined and further described herein and at least one pharmaceutically acceptable excipient. In one aspect, the pharmaceutical composition is a vaccination composition for a human and/or animal subject.

The present invention also encompasses a kit comprising an antibody or antigen-binding fragment thereof according to the invention as defined and further described herein and a container.

In one aspect, the present invention is also directed to the antibody or antigen-binding fragment thereof according to the invention as defined and further described herein, the pharmaceutical composition as described herein and the kit for use as a medicament, preferably for use as a vaccine.

In another aspect, the present invention is also directed to the antibody or antigen-binding fragment thereof according to the invention as defined and further described herein, the pharmaceutical composition as described herein and the kit for use in the treatment or prevention of a disease caused by SARS-related coronavirus in human or animal subjects, preferably for use in the treatment or prevention of a disease caused by SARS-related coronavirus 2 (SARS-CoV-2) in human or animal subjects.

In one aspect, the present invention is directed to the antibody or antigen-binding fragment thereof according to the invention as defined and further described herein, the pharmaceutical composition as described herein and the kit for use in prevention of infection of a human and/or animal subject with SARS-related coronavirus, preferably of infection of a human and/or animal subject with SARS-related coronavirus 2 (SARS-CoV-2).

In another aspect, the present invention is directed to a method of treating or preventing a SARS-related coronavirus infection or reducing the severity of disease in a human and/or animal subject comprising administering a therapeutically effective amount of at least one antibody and/or antigen-binding fragment thereof as described herein to said subject, preferably wherein the SARS-related coronavirus is SARS-CoV-2.

In one aspect of the invention, an antibody and/or antigen-binding fragment thereof according to the invention is administered to a patient in need thereof by intravenous injection or infusion, subcutaneous injection, intramuscular injection, or inhalative application, preferably by intravenous injection.

In a preferred embodiment, the antibody or antigen-binding fragment thereof is administered by intravenous infusion at an absolute dose of up to 4000 mg, preferably up to 2000 mg, more preferably up to 1200 mg, even more preferably up to 600 mg, even more preferably up to 300 mg, particularly preferably up to 150 mg.

The dosage of an antibody or antigen-binding fragment thereof of the invention to be administered to a subject can further vary depending on such things as the severity of the symptoms exhibited as well as the age, sex, and health of the subject.

In another aspect of the invention, an antibody according to the invention is administered to a patient in need thereof by inhalative application. In a preferred embodiment, the antibody is administered by inhalative application, wherein it is provided in a liquid pharmaceutical composition which is nebulized by a mesh nebulizer or a jet nebulizer prior to administration.

A pharmaceutical composition of the invention is formulated to be compatible with its intended route of administration. Examples of routes of administration include, but are not limited to, parenteral, e.g., intravenous, intradermal, subcutaneous, oral, intranasal (e.g., inhalation and inhaled through the mouth), transdermal (e.g., topical), transmucosal, and rectal administration.

In a specific embodiment, the composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

The methods of the invention may comprise pulmonary administration, e.g., by use of an inhaler or nebulizer, of a composition formulated with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entireties.

The methods of the invention may also comprise administration of a composition formulated for parenteral administration by injection (e.g., by bolus injection or continuous infusion). The pharmaceutical formulation of the present invention may be provided in liquid form or may be provided in lyophilized form.

In one aspect, the present invention relates to a nucleic acid encoding an antibody or antigen-binding fragment thereof as described herein. In another aspect, the present invention relates to an expression vector comprising the nucleic acid as described herein in functional association with an expression control sequence.

In another aspect, the present invention relates to a host cell comprising a nucleic acid as described herein. In one aspect, the present invention relates to a host cell comprising an expression vector as described herein.

In another aspect, the present invention relates to a method of production of an antibody or antigen-binding fragment as described herein, comprising
(a) cultivating a host cell as described herein under conditions allowing expression of the antibody or antigen-binding fragment thereof, and
(b) recovering the antibody or antigen-binding fragment thereof.

In another embodiment, the present invention relates to an antibody or antigen-binding fragment thereof against SARS-related coronavirus 2 (SARS-CoV-2) as described herein for use in medicine in combination with at least one further antibody directed against SARS-related coronavirus 2 (SARS-CoV-2), wherein said further antibody has a different binding specificity.

In another aspect, the present invention is also directed to the use of the antibody or antigen-binding fragment thereof according to the invention or a pharmaceutical composition of the invention in the manufacture of a medicament for treatment of a disease caused by SARS-related coronavirus in human or animal subjects, preferably for treatment or prevention of COVID-19 in human or animal subjects.

All embodiments of the present invention as described herein are deemed to be combinable in any combination, unless the skilled person considers such a combination to not make any technical sense.

### Examples

### SARS-CoV-2 infected individuals and sample collection

Blood samples were collected from donors who gave their written consent under the protocols 20-1187 and 16-054, approved by the Institutional Review Board (IRB) of the University of Cologne.

### Processing of serum, plasma and whole blood samples

Blood samples were collected in Heparin syringes or EDTA monovette tubes (Sarstedt) and fractionated into plasma and peripheral blood mononuclear cell (PBMC) by density gradient centrifugation using Histopaque-1077 (Sigma). Plasma aliquots were stored at -80°C until use. Serum was collected from Serum-gel tubes (Sarstedt) by centrifugation and stored at -80°C until use. PBMCs were stored at -150°C until use.

### Isolation of IgGs from serum and plasma samples

For the isolation of total IgG, 0.5-1 mL plasma was heat inactivated for 45 minutes at 56°C and incubated with Protein G Sepharose 4 Fast Flow beads (GE Healthcare) in PBS (Gibco) at 4°C overnight. On the next day, the beads were washed with PBS (Gibco) on chromatography columns (BioRad) and Protein G-bound IgG was eluted using 0.1M Glycine pH=3 and immediately buffered in 1M Tris pH=8. Buffer exchange to PBS (Gibco) was performed using 30 kDa cut-off Amicon Ultra-15 columns (Millipore) and the purified IgG was stored at 4°C.

### Cell lines

VeroE6 cells, HEK-293T cells and 293T-ACE2 cells were maintained in DMEM (Gibco) containing 10% FBS, 1% PS, 1mM L-Glutamine and 1mM Sodium pyruvate. Cells were grown on tissue culture treated dishes in a T75 flask (Sarstedt) at 37°C and 5% CO₂. 293-6E cells were maintained at 37°C and 6% CO₂ in FreeStyle Expression Medium (Thermo Fisher) and kept under constant shaking at 110-120 rpm.

### Single B cell sorting

The SARS-CoV-2 variant called HexaPro, which has a pre-fusion spike confirmation (Hsieh, C.L. et al. (2020). Science 369, 1501-1505) was used as bait protein for sorting spike-specific B cells from elite neutralizers using a 2-color sorting strategy. The HexaPro protein was labelled using the DyLight 488 or Dylight 650 antibody labeling kits (Thermo Fisher) as per the manufacturer's protocol. B cells were enriched from the peripheral blood mononuclear cell (PBMC) fraction using the CD19 Microbeads kit (Miltenyi Biotec) as per manufacturer's protocol.

Enriched B cells were stained with antibodies against human CD20 (AF700 labeled; Clone 2H7; BD Biosciences catalog #560631) and human IgG (PE labeled; Clone G18-145; BD Biosciences catalog #555787), DAPI (Thermo Fisher catalog # D1306), HexaPro-Dylight-488 and HexaPro-Dylight-650. B cells that were DAPI-negative, CD20-positive, IgG-positive, HexaPro-Dylight-488-positive and HexaPro-Dylight-650-positive were sorted onto 96 well plates containing sorting buffer comprised of 0.2 µl RNAsin (40U/µl Promega), 0.1 µl RNAseOut (40 U/µl Thermo Fisher), 0.2 µl 10X PBS, 0.4 µl DTT (100mM Promega) and 3.1 µl Nuclease free H₂O (Thermo Fisher). Sorts were done on a BD FACSAria^{™} Fusion cell sorter (Becton Dickinson) and sorted cells were frozen at -80°C until further processing.

### Single cell cDNA production and PCR

Sorted single B cells were lysed at 65°C for 1 min with 0.75 µl Random Hexamer Primer (200 ng/µl Thermo Fisher), 0.5 µl NP-40 (10% Thermo Fisher), 0.15 µl RNAseOUT (100mM Thermo Fisher) and 5.6 µl Nuclease-free H₂O (Thermo Fisher). Thereafter, 2 µl Nuclease-free H₂O (Thermo Fisher), 3 µl 5X RT Buffer (Invitrogen), 0.5 µl dNTPs (25mM Thermo Fisher), 1 µl DTT (100mM Invitrogen), 0.1 µl RNAsin (40 U/µl Promega), 0.1 µl RNaseOUT (40 U/µl Thermo Fisher) and 0.25 µl Superscript IV (200 U/µl Invitrogen) were added and reverse transcription performed by incubating at RT for 10 min, 42°C for 10 min, 25°C for 10 min, 50°C for 10 min and 94°C for 5 min.

Individual antibody sequences were amplified using semi-nested PCR. Heavy, kappa and lambda chains were simultaneously amplified in the 1^{st} PCR using Platinum Taq^{™} DNA polymerase (Thermo Fisher) using the 5' oPR-IGHV, oPR-IGKV and oPR-IGLV primer mix (Kreer, C. et al. (2020). J Immunol Methods 480, 112752) along with the 3' Cg-RT (Ozawa, T. et al. (2006). Biotechniques 40, 469-470, 472, 474 passim), 3' Cκ 543 (Tiller, T. et al. (2008). J Immunol Methods 329, 112-124) and 3' Cκ 494 (Tiller et al., 2008).

1^{st} PCR was run at 94°C for 1 min, 50 cycles of 94°C for 30 sec, 57°C for 30 sec, 72°C for 55 sec and final extension at 72°C for 5 min.

In the 2^{nd} PCR, the antibody chains were amplified in separate reaction using primer pairs of 5' oPR-IGHV + 3' IgG internal (Tiller et al., 2008) (heavy chain), 5' oPR-IGKV + 3' Cκ 494 (kappa chain) and 5' oPR-IGLV + 3' Xhol Cλ (Tiller et al., 2008) (lambda chain). 2^{nd} PCR was run at 94°C for 1 min, 50 cycles of 94°C for 30 sec, 57°C for 30 sec, 72°C for 45 sec and final extension at 72°C for 5 min. 2^{nd} PCR products were sequenced by Sanger sequencing for subsequent sequence analysis.

### Antibody sequence analysis

B cell sequences with a minimum length of 240 nucleotides and a mean Phred score of >=28 amino acids were annotated with IgBLAST (Ye, J. et al. (2013). Nucleic Acids Res 41, W34-40) and trimmed from Framework region (FWR) 1 to th end of the J gene of the variable region. Base calls with Phred score of <16 were masked and sequences with >15 masked nucleotides, frameshifts or stop codons were excluded from further analyses.

From these productive sequences, clonality was analyzed by grouping identical V genes, and the pairwise Levenshtein distance of their CDRH3 was determined. Individual sequences were grouped into clones when the shared the same V gene and the minimal CDRH3 identity was 75%. Sequences that remained after 10 rounds of randomized input were designated as being non-clonal. CDRH3 length was calculated based on IMGT numbering and % of sequences represent sequences with a particular V-gene out of all sequences within an individual.

### Antibody cloning for protein synthesis

Cloning of single B-cell-derived antibodies was done according to a previously published protocol (Gieselmann, L. et al. (2021). Nat Protoc 16, 3639-3671). The 1^{st} PCR product was used to amplify the variable regions for each antibody chain in separate reactions using the 5' primers SLIC-oPR-IGHV, SLIC-oPR-IGKV and SLIC-oPR-IGLV (Kreer et al., 2020) and 3' primers SLIC_IgG_HC_rev, SLIC_KC_rev and SLIC_LC_rev (Tiller et al., 2008) for heavy, kappa and lambda chain respectively.

PCR was performed using the Q5 Hot Start High Fidelity DNA Polymerase (NEB) and run at 98°C for 30 sec, 35 cycles of 98°C for 10 sec, 72°C for 45 sec and final extension at 72°C for 2 min. The PCR product was purified using the NucleoSpin^{®} 96 PCR Clean-up kit (Macherey-Nagel) as per the manufacturer's protocol and cloned into the respective plg expression vectors (plgG1, plgK, or plgL) by restriction digest and SLIC assembly (von Boehmer, L. et al. (2016). Nat Protoc 11, 1908-1923).

### Antibody synthesis

Antibodies were produced by transfection of 293-6E cells (National Research Council Canada) using branched polyethylenimine (PEI) 25kDa (Sigma-Aldrich) with 0.5 µg heavy chain plasmid and 0.5 µg light chain plasmid per 1 ml 293-6E culture. Cells were maintained at 37°C and 6% CO₂ FreeStyle 293 Expression Medium (Thermo Fisher) and 0.2% Penicillin/Streptomycin at 110-120 rpm. 7 days post transfection, cells were centrifuged and the cell culture supernatant was harvested, filtered with a 0.45 µm Nalgene Rapid Flow filter (Thermo Fisher) and incubated overnight at 4°C with Protein G Sepharose 4 Fast Flow (GE Healthcare) overnight.

Antibody-bound Sepharose beads were washed with PBS (Gibco) on chromatography columns (BioRad) and antibodies were eluted using 0.1 M Glycine pH=3 and immediately buffered in 1 M Tris pH=8. Thereafter, buffer exchange to PBS (Gibco) was performed using 50 kDa Amicon Ultra-15 spin columns (Millipore) and the antibodies were stored at 4°C.

### Cloning of SARS-CoV-2 spike variants

The codon optimized SARS-CoV-2 Wu01 spike (Hoffmann, M. et al. (2020). Cell 181, 271-280.e8) (isolate EPI_ISL_406716) was cloned into pCDNA^{™}3.1/V5-HisTOPO vector (Invitrogen). The D614G (B.1), 69-70 deletion and RBD mutants were generated by introducing the corresponding amino acid mutations using the Q5^{®} Site-Directed Mutagenesis Kit (NEB) and per manufacturer's protocol.

SARS-2-S RBD variants were generated in the B.1 background and included R346S; Q414H; K417E; N439K; N440K; K444Q; V445A; G446V; Y453F; G476S; S477N; T478K; E484K; F486V; F490S; Q493R; Q493K; S494P and N501Y. SARS-CoV-2 spike variants B1.1.7, B1.351, B.1.429, B.1.617 and B.1.617.2 were generated by introducing the corresponding amino acid mutations (see Figure 5) using PCR and HiFi assembly (NEBuilder^{®} HiFi DNA Assembly Kit, New England Biolabs) of overlapping segments as per manufacturer's protocol.

### Production and titration of pseudovirus particles

Pseudovirus particles were generated by co-transfection of individual plasmids encoding HIV-1 Tat, HIV-1 Gag/Pol, HIV-1 Rev, luciferase followed by an IRES and ZsGreen (reagents described in Crawford, K. H. D. et al. (2020). Viruses 12, 513), and the spike protein of SARS-CoV-2, SARS-CoV-1 or WIV-1. For production of pseudoviruses, HEK 293T cells were transfected with the pseudovirus-encoding plasmids using FuGENE^{®} 6 Transfection Reagent (Promega) as per the manufacturer's protocol. At 48 h and 72 h post transfection, cell debris was centrifugued and the virus culture supernatant was harvested and stored at -80°C until use.

Each virus batch was titrated by infecting 293T-ACE2 cells (293T cells expressing the human ACE2 receptor) and after a 48-hour incubation period at 37°C and 5% CO₂, luciferase activity was determined after addition of luciferin/lysis buffer (10 mM MgCl2, 0.3 mM ATP, 0.5 mM Coenzyme A, 17 mM IGEPAL (all Sigma-Aldrich), and 1 mM D-Luciferin (GoldBio) in Tris-HCL) using a microplate reader (Berthold). Pseudovirus dilutions resulting in relative luminiscence unit (RLU) differences of approximately 1000-fold between infected cells versus non-infected cells were used for pseudovirus neutralization assays.

### Pseudovirus neutralization assay to determine IgG neutralizing activity

For testing neutralizing activity of IgG (including monoclonal antibodies), 3-fold dilution series of IgG were prepared in neutralization assay medium (DMEM supplementend with 10% FBS, 100 U/ml penicillin, 100 U/ml streptomycin, 1 mM L-glutamine, and 1 mM sodium pyruvate). Diluted IgG was co-incubated with pseudovirus supernatants, produced and titrated as described above (section *"Production and titration of pseudovirus particles*"), for 1 hour at 37°C and 5% CO₂ in a total volume of 100 µL in a 96-well plate. The dilution series had a starting concentration (referring to the concentration during co-incubation of IgG and pseudovirus) of 10 µg/ml for monoclonal antibodies and 750 µg/ml for purified serum or plasma IgG. Subsequently, 1.25x10⁴ cells of 293T-ACE2 cells (cells described in Crawford et al., 2020), supplemented with polybrene at a final concentration of 5 µg/ml, were added to each well.

Following a 48-hour incubation at 37°C and 5% CO₂, luciferase activity was determined using the luciferin/lysis buffer reagents described above (section *"Production and titration of pseudovirus particles"*). Prior to addition of 100 µl luciferin/lysis buffer, 50 µl of cell culture supernatant was removed. After 2 min of lysis and mixing by pipetting, 150 µl of supernatant was transferred to a separate 96-well plate to determine RLUs on a microplate reader (measured 1 second per well).

After subtracting the average background RLUs of non-infected cells (wells containing cells that were not incubated with pseudovirus), the 50% inhibitory concentration (IC₅₀) was determined as the IgG concentrations resulting in a 50% RLU reduction compared to the average of untreated virus control wells (wells containing cells and psedovirus without any IgG).

IgG IC₅₀ values were calculated in GraphPad Prism 7.0 by using a non-linear fit model to plot an agonist vs. normalized dose response curve with variable slope using the least squares fitting method. A Y-value of 50% was used to calculate the corresponding X-value or IC₅₀ based on this dose response curve. Each IgG sample was measured in two independent experiments on different days and the average IC₅₀ values were reported.

### SARS-CoV-2 authentic virus neutralization assay

Authentic SARS-CoV-2 (COV2-P3) was previously grown out from a SARS-CoV-2 positive swab sample using VeroE6 cells as previously described (Vanshylla et al., 2021, Cell host & Microbe, 29(6):917-929.e4). For the neutralization assay, 5-fold serial dilutions of monoclonal IgGs in DMEM containing 2% FBS, 1% PS, 1mM L-Glutamine and 1mM Sodium pyruvate were prepared and co-incubated with the virus (1000-2000 TCID₅₀) for 1 hour at 37°C prior to addition of VeroE6 cells in DMEM (Gibco) containing 2% FBS, 1% PS, 1mM L-Glutamine and 1 mM Sodium pyruvate. The dilution series had a starting concentration (referring to the concentration during the co-incubation of IgG and virus) of 10 µg/ml for monoclonal antibodies.

After 4 days of incubation at 37°C and 5% CO₂, neutralization was analyzed by observing cytopathic effects (CPE) using a brightfield microscope and the antibody concentration in highest dilution well with no CPE was noted to be the IC₁₀₀ for the antibody. All samples were measured in two independent experiments on separate days and the average IC₁₀₀ from all measurements has been reported.

### Hep-2 autoreactivity assay

HEp-2 cell autoreactivity assay was performed as per manufacturer's protocol using the NOVA Lite Hep-2 ANA kit (Inova Diagnostics) using 100 µg/ml of monoclonal antibody in DPBS. As a positive control, the Hep-2 cell-recative HIV-1 neutralizing antibody 4E10 was included. Images were acquired using a Leica DMI3000 B microscope with 15 ms exposer and a gain of 10. Images were processed in Adobe Photoshop and assembled in Adobe Illustrator CC 2018^{®}. None of the antibodies of the present invention which were tested in this assay (R200-1B9, R207-2F11, R40-1G8, R568-2G5, R568-2B11, R207-2G4, R40-1C8, R568-2B9, R568-1B3, R568-2E1, R568-1G9 and R121-1F1) demonstrated autoreactivity in this assay (data not shown).

A summary of the results obtained with the antibodies of the invention is shown in Figures 2, 3, 4 and 6

## Claims

1. Antibody or antigen-binding fragment thereof directed against SARS-related coronavirus, wherein the antibody or antigen-binding fragment thereof comprises the combination of the heavy chain CDR1 to CDR3 and the light chain CDR1 to CDR3 amino acid sequence of one antibody selected from the group comprising:
R200-1B9 (having a CDR-H1 amino acid sequence of SEQ ID No. 27, a CDR-H2 amino acid sequence of SEQ ID No. 28, a CDR-H3 amino acid sequence of SEQ ID No. 29, a CDR-L1 amino acid sequence of SEQ ID No. 30, a CDR-L2 amino acid sequence of SEQ ID No. 31, a CDR-L3 amino acid sequence of SEQ ID No. 32),
R207-2F11 (having a CDR-H1 amino acid sequence of SEQ ID No. 33, a CDR-H2 amino acid sequence of SEQ ID No. 34, a CDR-H3 amino acid sequence of SEQ ID No. 35, a CDR-L1 amino acid sequence of SEQ ID No. 36, a CDR-L2 amino acid sequence of SEQ ID No. 37, a CDR-L3 amino acid sequence of SEQ ID No. 38),
R40-1G8 (having a CDR-H1 amino acid sequence of SEQ ID No. 39, a CDR-H2 amino acid sequence of SEQ ID No. 40, a CDR-H3 amino acid sequence of SEQ ID No. 41, a CDR-L1 amino acid sequence of SEQ ID No. 42, a CDR-L2 amino acid sequence of SEQ ID No. 43, a CDR-L3 amino acid sequence of SEQ ID No. 44),
R568-2G5 (having a CDR-H1 amino acid sequence of SEQ ID No. 45, a CDR-H2 amino acid sequence of SEQ ID No. 46, a CDR-H3 amino acid sequence of SEQ ID No. 47, a CDR-L1 amino acid sequence of SEQ ID No. 48, a CDR-L2 amino acid sequence of SEQ ID No. 49, a CDR-L3 amino acid sequence of SEQ ID No. 50),
R568-2B11 (having a CDR-H1 amino acid sequence of SEQ ID No. 51, a CDR-H2 amino acid sequence of SEQ ID No. 52, a CDR-H3 amino acid sequence of SEQ ID No. 53, a CDR-L1 amino acid sequence of SEQ ID No. 54, a CDR-L2 amino acid sequence of SEQ ID No. 55, a CDR-L3 amino acid sequence of SEQ ID No. 56),
R207-2G4 (having a CDR-H1 amino acid sequence of SEQ ID No. 57, a CDR-H2 amino acid sequence of SEQ ID No. 58, a CDR-H3 amino acid sequence of SEQ ID No. 59, a CDR-L1 amino acid sequence of SEQ ID No. 60, a CDR-L2 amino acid sequence of SEQ ID No. 61, a CDR-L3 amino acid sequence of SEQ ID No. 62),
R40-1C8 (having a CDR-H1 amino acid sequence of SEQ ID No. 63, a CDR-H2 amino acid sequence of SEQ ID No. 64, a CDR-H3 amino acid sequence of SEQ ID No. 65, a CDR-L1 amino acid sequence of SEQ ID No. 66, a CDR-L2 amino acid sequence of SEQ ID No. 67, a CDR-L3 amino acid sequence of SEQ ID No. 68),
R568-2B9 (having a CDR-H1 amino acid sequence of SEQ ID No. 69, a CDR-H2 amino acid sequence of SEQ ID No. 70, a CDR-H3 amino acid sequence of SEQ ID No. 71, a CDR-L1 amino acid sequence of SEQ ID No. 72, a CDR-L2 amino acid sequence of SEQ ID No. 73, a CDR-L3 amino acid sequence of SEQ ID No. 74),
R568-1B3 (having a CDR-H1 amino acid sequence of SEQ ID No. 75, a CDR-H2 amino acid sequence of SEQ ID No. 76, a CDR-H3 amino acid sequence of SEQ ID No. 77, a CDR-L1 amino acid sequence of SEQ ID No. 78, a CDR-L2 amino acid sequence of SEQ ID No. 79, a CDR-L3 amino acid sequence of SEQ ID No. 80),
R568-2E1 (having a CDR-H1 amino acid sequence of SEQ ID No. 81, a CDR-H2 amino acid sequence of SEQ ID No. 82, a CDR-H3 amino acid sequence of SEQ ID No. 83, a CDR-L1 amino acid sequence of SEQ ID No. 84, a CDR-L2 amino acid sequence of SEQ ID No. 85, a CDR-L3 amino acid sequence of SEQ ID No. 86),
R568-1G9 (having a CDR-H1 amino acid sequence of SEQ ID No. 87, a CDR-H2 amino acid sequence of SEQ ID No. 88, a CDR-H3 amino acid sequence of SEQ ID No. 89, a CDR-L1 amino acid sequence of SEQ ID No. 90, a CDR-L2 amino acid sequence of SEQ ID No. 91, a CDR-L3 amino acid sequence of SEQ ID No. 92),
R121-1F1 (having a CDR-H1 amino acid sequence of SEQ ID No. 93, a CDR-H2 amino acid sequence of SEQ ID No. 94, a CDR-H3 amino acid sequence of SEQ ID No. 95, a CDR-L1 amino acid sequence of SEQ ID No. 96, a CDR-L2 amino acid sequence of SEQ ID No. 97, a CDR-L3 amino acid sequence of SEQ ID No. 98),
R259-1B9 (having a CDR-H1 amino acid sequence of SEQ ID No. 99, a CDR-H2 amino acid sequence of SEQ ID No. 100, a CDR-H3 amino acid sequence of SEQ ID No. 101, a CDR-L1 amino acid sequence of SEQ ID No. 102, a CDR-L2 amino acid sequence of SEQ ID No. 103, a CDR-L3 amino acid sequence of SEQ ID No. 104).

2. Antibody or antigen-binding fragment thereof according to claim 1, wherein the antibody or antigen-binding fragment thereof comprises the combination of the variable region heavy chain amino acid sequence and of the variable region light chain amino acid sequence of one antibody selected from the group comprising:
R200-1B9 (having the variable region heavy chain amino acid sequence of SEQ ID No. 1 and the variable region light chain amino acid sequence of SEQ ID No. 2),
R207-2F11 (having the variable region heavy chain amino acid sequence of SEQ ID No. 3 and the variable region light chain amino acid sequence of SEQ ID No. 4),
R40-1G8 (having the variable region heavy chain amino acid sequence of SEQ ID No. 5 and the variable region light chain amino acid sequence of SEQ ID No. 6),
R568-2G5 (having the variable region heavy chain amino acid sequence of SEQ ID No. 7 and the variable region light chain amino acid sequence of SEQ ID No. 8),
R568-2B11 (having the variable region heavy chain amino acid sequence of SEQ ID No. 9 and the variable region light chain amino acid sequence of SEQ ID No. 10),
R207-2G4 (having the variable region heavy chain amino acid sequence of SEQ ID No. 11 and the variable region light chain amino acid sequence of SEQ ID No. 12),
R40-1C8 (having the variable region heavy chain amino acid sequence of SEQ ID No. 13 and the variable region light chain amino acid sequence of SEQ ID No. 14),
R568-2B9 (having the variable region heavy chain amino acid sequence of SEQ ID No. 15 and the variable region light chain amino acid sequence of SEQ ID No. 16),
R568-1B3 (having the variable region heavy chain amino acid sequence of SEQ ID No. 17 and the variable region light chain amino acid sequence of SEQ ID No. 18),
R568-2E1 (having the variable region heavy chain amino acid sequence of SEQ ID No. 19 and the variable region light chain amino acid sequence of SEQ ID No. 20),
R568-1G9 (having the variable region heavy chain amino acid sequence of SEQ ID No. 21 and the variable region light chain amino acid sequence of SEQ ID No. 22),
R121-1F1 (having the variable region heavy chain amino acid sequence of SEQ ID No. 23 and the variable region light chain amino acid sequence of SEQ ID No. 24),
R259-1B9 (having the variable region heavy chain amino acid sequence of SEQ ID No. 25 and the variable region light chain amino acid sequence of SEQ ID No. 26).

3. Antibody or antigen-binding fragment thereof according to any one of claims 1 or 2, wherein the amino acid sequences comprised are of one antibody selected from the group comprising R200-1B9, R207-2F11, R40-1G8, R568-2G5, R568-2B11, R207-2G4, R40-1C8, R568-2B9, and R568-1B3, preferably of one antibody from the group comprising R200-1B9, R207-2F11, R40-1G8, and R568-2G5.

4. Antibody or antigen-binding fragment thereof according to any one of claims 1 to 3, wherein the SARS-related coronavirus strain is severe acute respiratory syndrome coronavirus 2 (SARS-CoV-2).

5. Antibody or antigen-binding fragment thereof according to any one of claims 1 to 4, wherein the amino acid sequences of the CDRs or of the variable regions comprised therein are from an antibody which is able to neutralize each of the SARS-CoV-2 lineages B.1, B.1.1.7, B.1.351, B.1.429, B.1.617 and B.1.617.2 in a pseudovirus neutralization assay as described in the description with an IC50 of at most 0.4 µg/ml, preferably at most 0.1 µg/ml, more preferably at most 0.05 µg/ml, even more preferably at most 0.025 µg/ml, even more preferably at most 0.01 µg/ml, even more preferably at most 0.0025 µg/ml, particularly preferably at most 0.0015 µg/ml.

6. Antibody or antigen-binding fragment thereof according to any one of claims 1 to 5, wherein the amino acid sequences of the CDRs or of the variable regions comprised therein are from an antibody which is able to neutralize each of the SARS-CoV-2 spike escape mutants 69-70del, K417E, N439K, K444Q, V445A, G446V, L452R, Y453F, L455F, G476S, S477N, E484K, F486V, F490S, Q493R, Q493K, S494P, N501Y in a pseudovirus neutralization assay as described in the description with an IC50 of at most 1.3 µg/ml, preferably at most 0.5 µg/ml, more preferably at most 0.05 µg/ml, even more preferably at most 0.02 µg/ml, particularly preferably at most 0.01 µg/ml.

7. Antibody or antigen-binding fragment thereof according to any one of claims 1 to 6, wherein the amino acid sequences of the CDRs or of the variable regions comprised in the antibody or antigen-binding fragment thereof are taken from an antibody which is able to neutralize each of the emerging SARS-CoV-2 spike escape mutants R346S, Q414H, N440K, and T478K in a pseudovirus neutralization assay as described in the description with an IC50 of at most 0.0015 µg/ml.

8. Antibody or antigen-binding fragment thereof according to any one of claims 1 to 7, wherein the amino acid sequences of the CDRs or of the variable regions comprised therein are from an antibody which is able to neutralize the SARS-CoV-2 variants Wu-01, B.1, B.1.1.7, B.1.351, B.1.429, B.1.617 and B.1.617.2 and the SARS-CoV-2 escape mutants 69-70del, K417E, N439K, K444Q, V445A, G446V, L452R, Y453F, L455F, G476S, S477N, E484K, F486V, F490S, Q493R, Q493K, S494P, N501Y, R346S, Q414H, N440K, and T478K in a pseudovirus neutralization assay as described in the description across all variants and mutants with an average IC50 of at most 0.08 µg/ml, preferably of at most 0.025 µg/ml, more preferably of at most 0.01 µg/ml, even more preferably of at most 0.006 µg/ml, particularly preferably of at most 0.002 µg/ml.

9. Pharmaceutical composition comprising an antibody or antigen-binding fragment thereof according to any one of claims 1 to 8 and at least one pharmaceutically acceptable excipient, preferably wherein the pharmaceutical composition is a vaccination composition for a human subject.

10. Kit comprising an antibody or antigen-binding fragment thereof according to any one of claims 1 to 8 and a container.

11. Antibody or antigen-binding fragment thereof according to any one of claims 1 to 8, pharmaceutical composition according to claim 9, or kit according to claim 10 for use as a medicament.

12. Antibody or antigen-binding fragment thereof according to any one of claims 1 to 8, pharmaceutical composition according to claim 9, or kit according to claim 10 for use as a vaccine.

13. Antibody or antigen-binding fragment thereof according to any one of claims 1 to 8, pharmaceutical composition according to claim 9, or kit according to claim 10 for use in the treatment or prevention of a disease caused by SARS-related coronavirus in human subjects, preferably for use in the treatment or prevention of a disease caused by severe acute respiratory syndrome coronavirus 2 (SARS-CoV-2) in human subjects.

14. Antibody or antigen-binding fragment thereof according to any one of claims 1 to 8, pharmaceutical composition according to claim 9, or kit according to claim 10 for use in prevention of infection of a human subject with SARS-related coronavirus, preferably of infection of a human subject with severe acute respiratory syndrome coronavirus 2 (SARS-CoV-2).

15. Antibody or antigen-binding fragment thereof for use according to any one of claims 11 to 14, wherein the antibody or antigen-binding fragment thereof is administered by intravenous infusion, by inhalative application, by subcutaneous injection or intramuscular injection, preferably wherein the antibody or antigen-binding fragment thereof is administered at an absolute dose of up to 4000 mg, preferably up to 2400 mg, more preferably up to 1200 mg, even more preferably up to 600 mg, even more preferably up to 300 mg, particularly preferably up to 150 mg.

16. Nucleic acid encoding an antibody or antigen-binding fragment thereof according to any of claims 1 to 8.

17. An expression vector comprising the nucleic acid of claim 16 in functional association with an expression control sequence.

18. Host cell comprising a nucleic acid according to claim 16 or the expression vector according to claim 17.

19. Method of production of an antibody or antigen-binding fragment thereof according to any one of claims 1 to 8, comprising
(a) cultivating the host cell of claim 18 under conditions allowing expression of the antibody or antigen-binding fragment thereof, and
(b) recovering the antibody or antigen-binding fragment thereof.

20. The antibody of any of claims 1 to 8 for use in medicine in combination with at least one further antibody directed against SARS-related coronavirus 2 (SARS-CoV-2), wherein said further antibody has a different binding specificity.
